(19) Europäisches Patentamt
European Patent Office
Office européen des brevets

(11) **EP 4 703 766 A1**

(12) **EUROPEAN PATENT APPLICATION**
published in accordance with Art. 153(4) EPC

(43) Date of publication:
**04.03.2026 Bulletin 2026/10**

(21) Application number: **24796788.8**

(22) Date of filing: **09.04.2024**

(51) International Patent Classification (IPC):
*G01S 13/52* (2006.01)   *A61B 5/11* (2006.01)
*G01S 13/34* (2006.01)

(52) Cooperative Patent Classification (CPC):
**A61B 5/11; G01S 13/34; G01S 13/52**

(86) International application number:
**PCT/JP2024/014446**

(87) International publication number:
**WO 2024/225032 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.04.2023   JP 2023072698**

(71) Applicant: Kyocera Corporation
**Kyoto-shi, Kyoto 612-8501 (JP)**

(72) Inventors:
• **TAKAMATSU Tetsuya**
  **Kyoto-shi, Kyoto 612-8501 (JP)**
• **MURAKAMI Youhei**
  **Kyoto-shi, Kyoto 612-8501 (JP)**
• **KURODA Jun**
  **Kyoto-shi, Kyoto 612-8501 (JP)**
• **KASHIMA Yuu**
  **Kyoto-shi, Kyoto 612-8501 (JP)**
• **MATSUE Yudai**
  **Kyoto-shi, Kyoto 612-8501 (JP)**

(74) Representative: **Viering, Jentschura & Partner mbB**
**Patent- und Rechtsanwälte**
**Am Brauhaus 8**
**01099 Dresden (DE)**

(54) **ELECTRONIC DEVICE, METHOD FOR CONTROLLING ELECTRONIC DEVICE, AND PROGRAM**

(57)    An electronic device includes a signal processing unit. The signal processing unit is configured to detect a subject of monitoring. The detection is based on a transmission signal transmitted as a transmission wave and a received signal received as a reflected wave resulting from the transmission wave being reflected by the subject of monitoring. The signal processing unit is configured to generate a heart sound waveform of the subject of monitoring by using basis information pertaining to a heart sound waveform obtained in advance to perform non-negative matrix factorization of time-frequency analysis information calculated on the basis of the transmission wave and the reflected wave.

FIG. 2

**Description**

CROSS-REFERENCE TO RELATED APPLICATIONS

This application claims priority based on Japanese Patent Application No. 2023-72698 filed April 26, 2023, the entire disclosure of which is hereby incorporated by reference.

TECHNICAL FIELD

The present disclosure relates to an electronic device, a method of controlling an electronic device, and a program.

BACKGROUND OF INVENTION

In fields such as the automotive and related industries, for example, technologies for measuring values such as the distance between a vehicle and a given object are gaining importance. In particular, recent years have seen various research into radio detection and ranging (RADAR) technology, which measures values such as the distance to an obstacle or other object by transmitting radio waves, such as millimeter waves, and receiving reflected waves back from the object. The importance of technologies for measuring such distances and the like is expected to increase further with the development of technologies for assisting drivers with driving and technologies related to self-driving, in which driving is partially or fully automated.

Various technologies have been proposed to acquire and analyze sounds (biological sounds) emitted from a living body such as a human body. For example, Patent Literature 1 proposes a technology for separating and analyzing multiple types of sounds included in biological sounds such as respiratory sounds. Patent Literature 2 proposes a technology that may improve the precision of classifying, into multiple classes, time-series data in which a signal obtained by sensing is continuously generated. Patent Literature 3 proposes a signal analysis technique whereby basis spectra can be learned using an algorithm with guaranteed convergence.

CITATION LIST

PATENT LITERATURE

Patent Literature 1: International Publication No. 2015/145754
Patent Literature 2: Japanese Unexamined Patent Application Publication No. 2017-151872
Patent Literature 3: Japanese Unexamined Patent Application Publication No. 2018-136368

SUMMARY

An embodiment of the present disclosure is an electronic device including a signal processing unit. The signal processing unit is configured to detect a subject of monitoring on the basis of a transmission signal transmitted as a transmission wave and a received signal received as a reflected wave resulting from the transmission wave being reflected by the subject of monitoring. The signal processing unit is configured to generate a heart sound waveform of the subject of monitoring by using basis information pertaining to a heart sound waveform obtained in advance to perform non-negative matrix factorization of time-frequency analysis information calculated on the basis of the transmission wave and the reflected wave.

An embodiment of the present disclosure is an electronic device including a signal processing unit. The signal processing unit is configured to detect a subject of monitoring on the basis of a transmission signal transmitted as a transmission wave and a received signal received as a reflected wave resulting from the transmission wave being reflected by the subject of monitoring. The signal processing unit is configured to generate first time-frequency analysis information $Y1$ on the basis of the transmission wave and the reflected wave. The signal processing unit is configured to perform semi-supervised non-negative matrix factorization to calculate the first time-frequency analysis information $Y1$ as $Y1 = FG + HU$ using frequency basis matrices $F$ and $H$ and activation matrices $G$ and $U$, calculate the frequency basis matrix $F$ as a frequency basis matrix obtained from the reference heart sound waveform obtained in advance, and calculate the activation matrix $G$ using a prescribed cost function. The signal processing unit is configured to generate the heart sound waveform of the subject of monitoring on the basis of the product $FG$ of the frequency basis matrix $F$ and the activation matrix $G$.

An embodiment of the present disclosure is a method of controlling an electronic device. The method involves detecting a subject of monitoring on the basis of a transmission signal transmitted as a transmission wave and a received signal received as a reflected wave resulting from the transmission wave being reflected by the subject of monitoring. The method involves generating first time-frequency analysis information Y1 on the basis of the transmission wave and the reflected wave. The method involves performing semi-supervised non-negative matrix factorization to calculate the first time-frequency analysis information Y1 as Y1 = FG + HU using frequency basis matrices F and H and activation matrices G and U, calculate the frequency basis matrix F as a frequency basis matrix obtained from the reference heart sound waveform obtained in advance, and calculate the activation matrix G using a prescribed cost function. The method involves generating the heart sound waveform of the subject of monitoring on the basis of the product FG of the frequency basis matrix F and the activation matrix G.

An embodiment of the present disclosure is a program. The program causes an electronic device to perform a process. The process involves detecting a subject of monitoring on the basis of a transmission signal transmitted as a transmission wave and a received signal received as a reflected wave resulting from the transmission wave being reflected by the subject of monitoring. The process involves generating first time-frequency analysis information Y1 on the basis of the transmission wave and the reflected wave. The process involves performing semi-supervised non-negative matrix factorization to calculate the first time-frequency analysis information Y1 as Y1 = FG + HU using frequency basis matrices F and H and activation matrices G and U, calculate the frequency basis matrix F as a frequency basis matrix obtained from the reference heart sound waveform obtained in advance, and calculate the activation matrix G using a prescribed cost function. The process involves generating the heart sound waveform of the subject of monitoring on the basis of the product FG of the frequency basis matrix F and the activation matrix G.

BRIEF DESCRIPTION OF THE DRAWINGS

FIG. 1 is a diagram for describing how an electronic device according to an embodiment is used.
FIG. 2 is a function block diagram schematically illustrating a configuration of an electronic device according to an embodiment.
FIG. 3 is a diagram for describing the composition of a signal processed by an electronic device according to an embodiment.
FIG. 4 is a diagram for describing the processing of a signal by an electronic device according to an embodiment.
FIG. 5 is a diagram for describing the processing of a signal by an electronic device according to an embodiment.
FIG. 6 is a diagram for describing the processing of a signal by an electronic device according to an embodiment.
FIG. 7 schematically illustrates an example of the antenna arrangement and the operating principle of an antenna array of an electronic device according to an embodiment.
FIG. 8 illustrates an example of the antenna arrangement in an antenna array of an electronic device according to an embodiment.
FIG. 9 illustrates an example of the processing of a signal by an electronic device according to an embodiment.
FIG. 10 illustrates an example of the processing of a signal by an electronic device according to an embodiment.
FIG. 11 is a flowchart for describing a comparative example of operations by an electronic device according to an embodiment.
FIG. 12 is a flowchart for describing operations by an electronic device according to an embodiment.
FIG. 13 is a flowchart for describing operations by an electronic device according to an embodiment.
FIG. 14 uses pseudocode to illustrate semi-supervised non-negative matrix factorization processing by an electronic device according to an embodiment.
FIG. 15 uses heart sound waveforms and spectrograms to illustrate an example of processing by an electronic device according to an embodiment.
FIG. 16 illustrates an example of abnormality detection through singular spectrum transformation by an electronic device according to an embodiment.
FIG. 17 uses pseudocode to illustrate singular spectrum transformation processing by an electronic device according to an embodiment.
FIG. 18 is a flowchart for describing operations by an electronic device according to an embodiment.
FIG. 19 illustrates an example of a result of signal processing by an electronic device according to an embodiment.
FIG. 20 is a diagram for describing signal processing by an electronic device according to an embodiment.
FIG. 21 is a flowchart for describing operations by an electronic device according to an embodiment.
FIG. 22 illustrates an example of a result of signal processing by an electronic device according to an embodiment.

DESCRIPTION OF EMBODIMENTS

The ability to detect weak vibrations such as a heartbeat with good precision by transmitting and receiving radio waves, such as millimeter waves for example, could be useful in a wide variety of fields. An objective of the present disclosure is to provide an electronic device, a method of controlling an electronic device, and a program with which a heartbeat can be detected with good precision by transmitting and receiving radio waves. According to an embodiment, it is possible to provide an electronic device, a method of controlling an electronic device, and a program with which a heartbeat can be detected with good precision by transmitting and receiving radio waves. The following describes an embodiment with reference to the drawings.

In the present disclosure, an "electronic device" may be a device driven by electric power. A "user" may be an entity (typically a human being) or an animal that uses an electronic device according to an embodiment and/or a system including the electronic device. The user may include an entity that monitors a human being or other subject by using an electronic device according to an embodiment. The "subject" may be an entity (subject of monitoring, such as a human being or an animal, for example) to be monitored using an electronic device according to an embodiment. The user may also include the subject.

In the present disclosure, "heartbeat" may refer to the pulsation of the heart. "Pulsation" may refer to a rhythmic contraction movement performed by the heart. The heart may be a human heart or an animal heart. The "heartbeat" and the "heart sound" may be the "heartbeat" and the "heart sound" of a human or an animal.

In the present disclosure, "heart rate" refers to the number of times the heart pulsates in a prescribed period of time. For example, the heart rate may refer to the number of pulsations per minute. Pulsations occur in the arteries when the heart pumps blood. Accordingly, the number of pulsations by the arteries may also be referred to as the "pulse rate" or simply the "pulse".

In the present disclosure, "heart sound" may refer to the sound of a beating heart. That is, the heart sound may refer to the sound that occurs when the heart contracts and dilates. The "heart sound" may consist of a low, long first sound based on ventricular muscle tension, mitral valve closure, initiation of blood ejection into the arteries, and/or acceleration of blood flow, followed by a high, short second sound derived from aortic valve closure and/or pulmonary valve closure.

In the present disclosure, the heart sound is not necessarily limited to physical sounds based on air vibrations, and may also mean the vibrations themselves caused by the beating (pulsation) of the heart. For example, in the present disclosure, the heartbeat may be assumed to imply a vibrating source, and the heart sound may be assumed to imply the vibrations themselves caused by the vibrating source. In the present disclosure, the heartbeat may also be assumed to imply the heart sound, depending on the situation.

An electronic device according to an embodiment can detect the heartbeat of a human being or other subject present in the surroundings of the electronic device. Accordingly, anticipated situations in which an electronic device according to an embodiment is used may be, for example, specific facilities or the like used by people engaged in social activities, such as companies, hospitals, nursing homes, schools, sports gyms, and nursing care facilities. For example, it is extremely important for a company to grasp and/or manage the health status of its employees and the like. Similarly, it is extremely important for a hospital to grasp and/or manage the health status of its patients, medical personnel, and the like, and for a nursing home to grasp and/or manage the health status of its residents, staff, and the like. The situations in which an electronic device according to an embodiment is used are not limited to facilities such as companies, hospitals, and nursing homes as described above, and may be any given facility where it is desirable to grasp and/or manage the health status of a subject. The any given facility may also include a non-commercial facility, such as the home of a user. The situations in which an electronic device according to an embodiment is used are not limited to indoors and may also be outdoors. For example, the situations in which an electronic device according to an embodiment is used may also be inside means of transportation such as trains, buses, airplanes, and the like, as well as stations, boarding areas, and the like. The situations in which an electronic device according to an embodiment is used may also be a means of transportation such as an automobile, aircraft, or ship, a hotel, the home of a user, or the living room, bath, toilet, bedroom, or the like in a home. The situations in which an electronic device according to an embodiment is used may also be when measuring the heartbeats of animals such as cows, pigs, or other livestock at zoos, ranches, farms, and the like.

In a nursing facility, for example, an electronic device according to an embodiment may be used for the purpose of detecting or monitoring the heartbeat of a subject such as a person in need of nursing or care. If an abnormality is found in the heartbeat of a subject such as a person in need of nursing or care, for example, an electronic device according to an embodiment may also issue a predetermined warning to the person in question and/or another person. Consequently, according to an electronic device according to an embodiment, the person in question and/or a staff member of a nursing care facility or the like may recognize that an abnormality is found in the pulse of a subject such as a person in need of nursing or care, for example. On the other hand, if an abnormality is not found (for example, the heartbeat is found to be normal) in the heartbeat of a subject such as a person in need of nursing or care, for example, an electronic device according to an embodiment may also issue a notification to that effect to the person in question and/or another person. Consequently, according to an electronic device according to an embodiment, the person in question and/or a staff member of a nursing care facility or the like may recognize that the pulse is normal for a subject such as a person in need of nursing or care, for example. An electronic device according to an embodiment may also use the heartbeat of a subject of monitoring to estimate the heartbeat interval (the RR interval (RRI) in an electrocardiogram), detection of distracted driving such as falling asleep at the wheel, and predictive signs thereof, estimation of level of alertness, estimation of level of fatigue, or identification of the subject of monitoring.

An electronic device according to an embodiment may also detect the pulse of an animal other than a human being as the subject. As an example, the electronic device according to an embodiment described hereinafter is described as detecting the pulse of a human being using a sensor based on a technology like millimeter-wave radar.

An electronic device according to an embodiment may be installed in any stationary object, and may also be installed in any moving object. An electronic device according to an embodiment can transmit a transmission wave from a transmitting antenna to the surroundings of the electronic device. An electronic device according to an embodiment can receive, from a receiving antenna, a reflected wave resulting from the transmission wave being reflected. At least one of the transmitting antenna or the receiving antenna may be provided in the electronic device, and may also be provided in a radar sensor, for example.

In the following, an electronic device according to an embodiment is described as stationary. An electronic device according to an embodiment may also be installed in a moving body such as an automobile, for example. By being installed in a moving body such as an automobile, for example, an electronic device according to an embodiment may detect the heartbeat and the like of an occupant on board the moving body. On the other hand, the subject (human being) whose pulse is to be detected by an electronic device according to an embodiment may be stationary, moving, or moving their body while remaining stationary. Like an ordinary radar sensor, an electronic device according to an embodiment can measure the distance and the like between the electronic device and an object in the surroundings of the electronic device in situations in which the object may move. An electronic device according to an embodiment can measure the distance and the like between the electronic device and an object even if the electronic device and the object are both stationary.

The following describes an electronic device according to an embodiment in detail, with reference to the drawings. First, an example of the detection of an object by an electronic device according to an embodiment will be described.

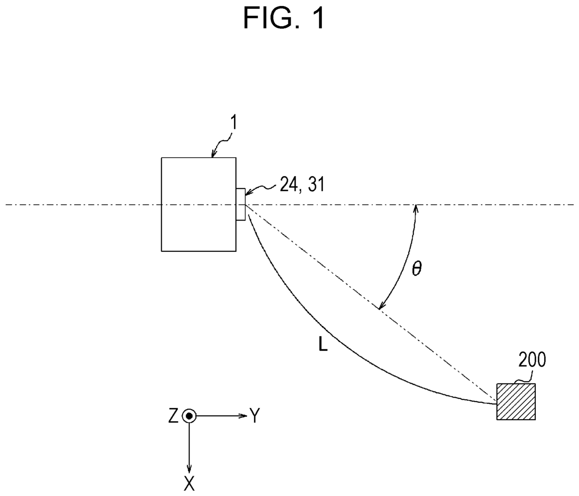

FIG. 1 is a diagram for describing an example of how an electronic device according to an embodiment is used. FIG. 1 illustrates an example of an electronic device provided with the functions of a sensor provided with a transmitting antenna and a receiving antenna according to an embodiment.

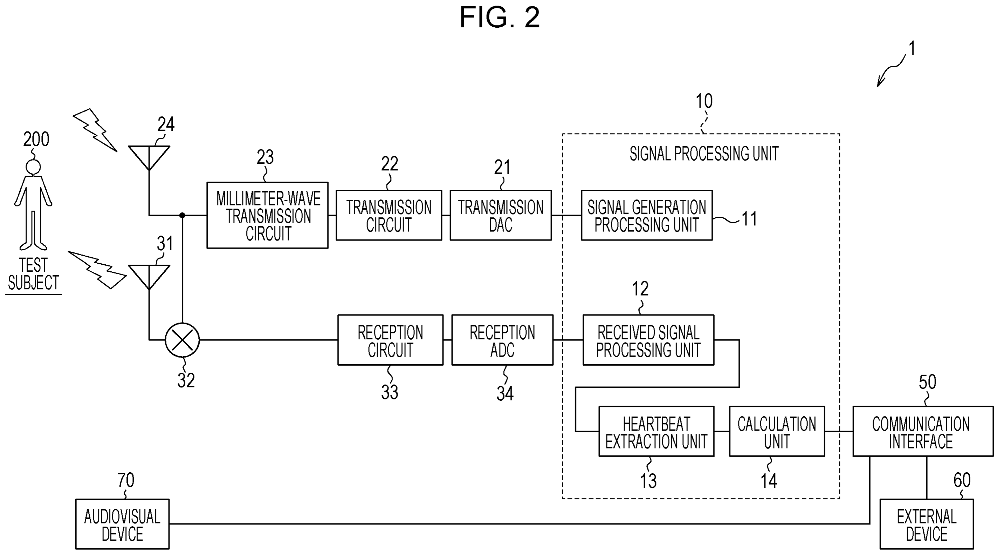

As illustrated in FIG. 1, in an embodiment, an electronic device 1 may be provided with a transmission unit and a reception unit. As described later, the transmission unit may be provided with a transmitting antenna 24. The reception unit may be provided with a receiving antenna 31. Specific configurations of the electronic device 1, the transmission unit, and the reception unit will be described later. For simplicity, FIG. 1 illustrates a situation in which the electronic device 1 is provided with the transmitting antenna 24 and the receiving antenna 31. The electronic device 1 may also include at least one other functional unit, as appropriate, such as at least a portion of a signal processing unit 10 (FIG. 2) included in the electronic device 1. The electronic device 1 may be provided with at least one other functional unit outside the electronic device 1, such as at least a portion of a signal processing unit 10 (FIG. 2) included in the electronic device 1. In FIG. 1, the electronic device 1 may be moving, but may also be stationary without moving.

The example illustrated in FIG. 1 illustrates in a simplified manner the transmission unit provided with the transmitting antenna 24 and the reception unit provided with the receiving antenna 31 in the electronic device 1. The electronic device 1 may also be provided with a plurality of transmission units and/or a plurality of reception units, for example. The transmission unit may be provided with a transmitting antenna 24 formed from a plurality of transmitting antennas. The reception unit may be provided with a receiving antenna 31 formed from a plurality of receiving antennas. The position where the transmission unit and/or reception unit are installed in the electronic device 1 is not limited to the position illustrated in FIG. 1, and may be another position, as appropriate. The number of transmission units and/or reception units may be any number equal to or greater than 1, according to various conditions (or requirements) such as the range and/or precision of heartbeat detection by the electronic device 1.

As described later, the electronic device 1 transmits an electromagnetic wave as a transmission wave from the transmitting antenna 24. For example, if a given object (for example, the subject 200 illustrated in FIG. 1) is present in the surroundings of the electronic device 1, at least a portion of the transmission wave transmitted from the electronic device 1 is reflected by the object to become a reflected wave. Such a reflected wave is then received by the receiving antenna 31 of the electronic device 1, for example, whereby the electronic device 1 can detect the subject as a target.

Typically, the electronic device 1 provided with the transmitting antenna 24 may be a radio detection and ranging (RADAR) sensor that transmits and receives radio waves. However, the electronic device 1 is not limited to a radar sensor. In an embodiment, the electronic device 1 may also be, for example, a sensor based on light detection and ranging (LIDAR) technology, also known as laser imaging detection and ranging, which involves light waves. Sensors such as these can be configured to include a patch antenna or the like. Since technologies such as RADAR and LIDAR are already known, a detailed description of these technologies may be simplified or omitted, as appropriate. In an embodiment, the electronic device 1 may also be a sensor based on a technology that detects objects by transmitting and receiving sonic or ultrasonic waves, for example.

The electronic device 1 illustrated in FIG. 1 receives from the receiving antenna 31 a reflected wave of a transmission wave transmitted from the transmitting antenna 24. With this arrangement, the electronic device 1 can detect a given subject 200 present within a given distance from the electronic device 1 as a target. For example, as illustrated in FIG. 1, the electronic device 1 can measure the distance L between the electronic device 1 and a given subject 200. The electronic device 1 can also measure the relative velocity between the electronic device 1 and a given subject 200. The electronic device 1 can also measure the direction (angle of arrival θ) from which the reflected wave from a given subject 200 arrives at the electronic device 1.

In FIG. 1, the XY plane may be defined as the plane substantially parallel to the ground, for example. In this case, the positive direction of the Z axis illustrated in FIG. 1 may indicate the vertically upward direction. In FIG. 1, the electronic device 1 may be installed on a plane parallel to the XY plane. In FIG. 1, the subject 200 may be standing on the ground substantially parallel to the XY plane, for example.

The subject 200 may be a human being or the like present in the surroundings of the electronic device 1, for example. The subject 200 may also be a living thing other than a human being, such as an animal, present in the surroundings of the electronic device 1, for example. As described above, the subject 200 may be moving, and may also be stopped or stationary. In the present disclosure, the object to be detected by the electronic device 1 includes inanimate things such as any object, as well as living things such as people, dogs, cats, horses, and other animals. The object to be detected by the electronic device 1 according to the present disclosure may also include a target, including a person, a thing, an animal, or the like, that is detected by radar technology. In the present disclosure, a target may include a person, a thing, an animal, or the like. The following description assumes that the object such as the subject 200 present in the surroundings of the electronic device 1 is a human being (or an animal). Hereinafter, the "subject 200" is also referred to as the "test subject 200", as appropriate. In the present disclosure, the target may also be the subject 200 above.

In FIG. 1, the ratio of the size of the electronic device 1 to the size of the subject 200 does not necessarily indicate the actual ratio. In FIG. 1, the transmitting antenna 24 of the transmission unit and the receiving antenna 31 of the reception unit are illustrated as being installed on the outside of the electronic device 1. However, in an embodiment, the transmitting antenna 24 of the transmission unit and/or the receiving antenna 31 of the reception unit may be installed at various positions in the electronic device 1. For example, in an embodiment, the transmitting antenna 24 of the transmission unit and/or the receiving antenna 31 of the reception unit may be installed inside the electronic device 1, and may not appear on the exterior of the electronic device 1.

The following describes a typical example in which the transmitting antenna of the electronic device 1 transmits radio waves in a frequency band such as millimeter waves (30 GHz or higher) or quasi-millimeter waves (for example, around 20-30 GHz). On the other hand, the transmitting antenna of the electronic device 1 may also transmit radio waves with a frequency bandwidth of 4 GHz, such as from 77 GHz to 81 GHz, for example. The transmitting antenna of the electronic device 1 may also transmit radio waves of high frequency (for example, 30 GHz to 300 GHz) in or above the millimeter-wave band.

FIG. 2 is a function block diagram schematically illustrating an example configuration of the electronic device 1 according to an embodiment. The following describes an example of the configuration of the electronic device 1 according to an embodiment.

Frequency-modulated continuous-wave radar (hereinafter referred to as FMCW radar) is often used to measure distances and the like using millimeter-wave radar. In FMCW radar, a transmission signal is generated by sweeping the frequencies of the radio waves to be transmitted. Accordingly, in a millimeter-wave FMCW radar that uses radio waves in the 79 GHz frequency band, for example, the frequencies of the radio waves to be used have a frequency bandwidth of 4 GHz, such as from 77 GHz to 81 GHz, for example. Radar in the 79 GHz frequency band is characterized by having a wider usable frequency bandwidth than other millimeter/quasi-millimeter wave radars, such as radars in the 24 GHz, 60 GHz, and 76 GHz frequency bands, for example. The following describes such an embodiment as an example.

The radar scheme of the FMCW radar used in the present disclosure may include a fast-chirp modulation (FCM) scheme that transmits chirp signals on a shorter period than usual. The signal that the electronic device 1 generates is not limited to a signal of the FMCW scheme. The signal that the electronic device 1 generates may also be a signal of any of various schemes other than the FMCW scheme. A transmission signal sequence stored in any storage unit may be different depending on these various schemes. For example, in the case of a radar signal of the FMCW scheme described above, a signal of increasing frequency and a signal of decreasing frequency at each time sample may be used. Known technologies can be applied, as appropriate, for the various schemes described above, and thus a more detailed description is omitted.

As illustrated in FIG. 2, in an embodiment, the electronic device 1 is provided with a signal processing unit 10. The signal processing unit 10 may be provided with a signal generation processing unit 11, a received signal processing unit 12, a heartbeat extraction unit 13, and a calculation unit 14. The heartbeat extraction unit 13 may execute processing to extract a micro-Doppler component, for example. The heartbeat extraction unit 13 may also execute processing to extract an envelope of the heart sound of the test subject 200. The calculation unit 14 may execute processing to calculate a heartbeat interval (RRI) of the test subject 200, for example. The calculation unit 14 may also execute processing to calculate the heartbeat of the test subject 200, for example. The calculation unit 14 may further execute processing to calculate a heart rate variability (HRV) of the test subject 200, for example. In this case, the calculation unit 14 may also execute processing to perform frequency analysis of time-series data on the extracted heartbeat interval of the test subject 200. The calculation unit 14 may also execute processing to calculate the heart rate variability of the test subject 200 on the basis of frequency analysis of time-series data on the heartbeat interval. The signal generation processing unit 11, received signal processing unit 12, heartbeat extraction unit 13, and calculation unit 14 will be further described later, as appropriate. In the present disclosure, the heart sound may refer to, for example, a chest vibration waveform observed directly by radar, or to chest vibrations. The heartbeat refers to the pulsations themselves of the heart. A heartbeat interval, heart rate, or the like may be calculated from the movement of the heartbeat. The heartbeat interval may refer to the time interval between a pulsation of the heart and the next pulsation of the heart.

In an embodiment, the electronic device 1 is provided with a transmission DAC 21, a transmission circuit 22, a millimeter-wave transmission circuit 23, and the transmitting antenna 24 as the transmission unit. In an embodiment, the electronic device 1 is provided with the receiving antenna 31, a mixer 32, a reception circuit 33, and a reception ADC 34 as the reception unit. In an embodiment, the electronic device 1 need not include at least one of the functional units illustrated in FIG. 2, and may also include a functional unit other than the functional units illustrated in FIG. 2. The electronic device 1 illustrated in FIG. 2 may be formed using a circuit configured in basically the same and/or similar way as a common radar using electromagnetic waves in the millimeter-wave band or the like. On the other hand, in the electronic device 1 according to an embodiment, the signal processing by the signal processing unit 10 may include processing different from the processing performed by a common radar of the related art.

In an embodiment, the signal processing unit 10 provided in the electronic device 1 can control operations by the electronic device 1 as a whole, including control of each of the functional units that make up the electronic device 1. In particular, the signal processing unit 10 performs various processing with respect to signals handled by the electronic device 1. To provide control and processing power for executing various functions, the signal processing unit 10 may include at least one processor, such as a central processing unit (CPU) or a digital signal processor (DSP). The signal processing unit 10 may be realized entirely with a single processor, with several processors, or with respectively discrete processors. The processor may be realized as a single integrated circuit. An integrated circuit is also referred to as an IC. The processor may be realized as a plurality of communicatively connected integrated circuits and discrete circuits. The processor may be realized on the basis of any of various other known technologies. In an embodiment, the signal processing unit 10 may be configured as a CPU (hardware) and a program (software) executed by the CPU, for example. The signal processing unit 10 may include a storage unit (memory) required for operations by the signal processing unit 10.

The signal generation processing unit 11 of the signal processing unit 10 generates a signal to be transmitted from the electronic device 1. In the electronic device 1 according to an embodiment, the signal generation processing unit 11 may generate a transmission signal such as a chirp signal (transmission chirp signal). In particular, the signal generation processing unit 11 may generate signals (linear chirp signals) whose frequency varies periodically and linearly. For example, the signal generation processing unit 11 may generate chirp signals whose frequency increases periodically and linearly from 77 GHz to 81 GHz over time. As another example, the signal generation processing unit 11 may generate a signal whose frequency periodically and linearly increases (up-chirp) from 77 GHz to 81 GHz and then decreases (down-chirp) over time. The signal that the signal generation processing unit 11 generates may also be preset in the signal processing unit 10, for example. The signal that the signal generation processing unit 11 generates may also be stored in advance in any storage unit or the like in the signal processing unit 10, for example. Since chirp signals used in technical fields such as radar are already known, a more detailed description is simplified or omitted, as appropriate. The signal generated by the signal generation processing unit 11 is supplied to the transmission DAC 21. For this reason, the signal generation processing unit 11 may be connected to the transmission DAC 21.

The transmission DAC (digital-to-analog converter) 21 functions to convert a digital signal supplied from the signal generation processing unit 11 to an analog signal. The transmission DAC 21 may include a common digital-to-analog converter. The signal converted to analog by the transmission DAC 21 is supplied to the transmission circuit 22. For this reason, the transmission DAC 21 may be connected to the transmission circuit 22.

The transmission circuit 22 functions to convert the signal converted to analog by the transmission DAC 21 to an intermediate frequency (IF) band. The transmission circuit 22 may include a common IF-band transmission circuit. The signal processed by the transmission circuit 22 is supplied to the millimeter-wave transmission circuit 23. For this reason, the transmission circuit 22 may be connected to the millimeter-wave transmission circuit 23.

The millimeter-wave transmission circuit 23 functions to transmit the signal processed by the transmission circuit 22 as a millimeter wave (RF wave). The millimeter-wave transmission circuit 23 may include a common millimeter-wave transmission circuit. The signal processed by the millimeter-wave transmission circuit 23 is supplied to the transmitting antenna 24. For this reason, the millimeter-wave transmission circuit 23 may be connected to the transmitting antenna 24. The signal processed by the millimeter-wave transmission circuit 23 is also supplied to the mixer 32. For this reason, the millimeter-wave transmission circuit 23 may also be connected to the mixer 32.

The transmitting antenna 24 is a plurality of transmitting antennas arranged into an array. In FIG. 2, the configuration of the transmitting antenna 24 is illustrated in a simplified manner. The transmitting antenna 24 transmits a signal processed by the millimeter-wave transmission circuit 23 to the outside of the electronic device 1. The transmitting antenna 24 may include a transmitting antenna array used in a common millimeter-wave radar.

In this way, in an embodiment, the electronic device 1 is provided with a transmitting antenna (transmitting antenna 24) and can transmit a transmission signal (transmission chirp signal, for example) as a transmission wave from the transmitting antenna 24.

As an example, suppose the case in which an object such as the test subject 200 is present in the surroundings of the electronic device 1, as illustrated in FIG. 2. In this case, at least a portion of the transmission wave transmitted from the transmitting antenna 24 is reflected by an object such as the test subject 200. The at least a portion of the transmission wave transmitted from the transmitting antenna 24 that is reflected by an object such as the test subject 200 may be reflected toward the receiving antenna 31.

The receiving antenna 31 receives a reflected wave. The reflected wave may refer to at least a portion of a transmission wave transmitted from the transmitting antenna 24 that is reflected by an object such as the test subject 200.

The receiving antenna 31 is a plurality of receiving antennas arranged into an array. In FIG. 2, the configuration of the receiving antenna 31 is illustrated in a simplified manner. The receiving antenna 31 receives a reflected wave resulting from a transmission wave transmitted from the transmitting antenna 24 being reflected. The receiving antenna 31 may include a receiving antenna array used in a common millimeter-wave radar. The receiving antenna 31 supplies a received signal received as a reflected wave to the mixer 32. For this reason, the receiving antenna 31 may be connected to the mixer 32.

The mixer 32 converts a signal (transmission signal) processed by the millimeter-wave transmission circuit 23 and a received signal received by the receiving antenna 31 to an intermediate frequency (IF) band. The mixer 32 may include a mixer used in a common millimeter-wave radar. The mixer 32 supplies a signal generated as a synthesized result to the reception circuit 33. For this reason, the mixer 32 may be connected to the reception circuit 33.

The reception circuit 33 functions to perform analog processing on a signal converted to an IF band by the mixer 32. The reception circuit 33 may include a common reception circuit that performs conversion to an IF band. The signal processed by the reception circuit 33 is supplied to the reception ADC 34. For this reason, the reception circuit 33 may be connected to the reception ADC 34.

The reception ADC (analog-to-digital converter) 34 functions to convert an analog signal supplied from the reception circuit 33 to a digital signal. The reception ADC 34 may include a common analog-to-digital converter. The signal converted to digital by the reception ADC 34 is supplied to the received signal processing unit 12 of the signal processing unit 10. For this reason, the reception ADC 34 may be connected to the signal processing unit 10.

The received signal processing unit 12 of the signal processing unit 10 functions to perform various processing on a digital signal supplied from the reception ADC 34. For example, the received signal processing unit 12 calculates the distance from the electronic device 1 to an object such as the test subject 200 on the basis of a digital signal supplied from the reception ADC 34 (distance measurement). The received signal processing unit 12 also calculates the relative velocity of an object such as the test subject 200 relative to the electronic device 1 on the basis of a digital signal supplied from the reception ADC 34 (velocity measurement). The received signal processing unit 12 further calculates the bearing angle of an object such as the test subject 200 as seen from the electronic device 1 on the basis of a digital signal supplied from the reception ADC 34 (angle measurement). Specifically, I/Q converted data may be inputted into the received signal processing unit 12. By accepting the input of such data, the received signal processing unit 12 performs a fast Fourier transform (2D-FFT) in each of the distance (range) and velocity directions. The received signal processing unit 12 may then perform false alarm suppression and fixed probability conversion by removing noise points through processing such as constant false alarm rate (CFAR). The received signal processing unit 12 can perform angle-of-arrival estimation for points that satisfy the CFAR criterion to obtain the position of an object such as the test subject 200. The information generated as a result of the distance measurement, velocity measurement, and angle measurement by the received signal processing unit 12 may be supplied to the heartbeat extraction unit 13.

The heartbeat extraction unit 13 extracts information related to heartbeat from information generated by the received signal processing unit 12. The operations for extracting information related to heartbeat by the heartbeat extraction unit 13 will be further described later. The information related to heartbeat extracted by the heartbeat extraction unit 13 may be supplied to the calculation unit 14.

The calculation unit 14 performs various arithmetic processing, computational processing, and/or the like on information related to heartbeat supplied from the heartbeat extraction unit 13. The arithmetic processing, computational processing, and/or the like by the calculation unit 14 will be further described later. Various information resulting from the arithmetic processing, computational processing, and/or the like by the calculation unit 14 may be supplied to, for example, a communication interface 50. For this reason, the calculation unit 14 and/or signal processing unit 10 may be connected to the communication interface 50. Various information resulting from the arithmetic processing, computational processing, and/or the like by the calculation unit 14 may also be supplied to another functional unit other than the communication interface 50.

The communication interface 50 is configured to include an interface that outputs information supplied from the signal processing unit 10 to, for example, an external device 60. The communication interface 50 may output information on at least one of the position, velocity, and angle of an object such as the test subject 200 as a controller area network (CAN) or other type of signal, for example, to the external device 60 or the like. For example, information on at least one of the position, velocity, and angle of an object such as the test subject 200 may be supplied to the external device 60 or the like via the communication interface 50. For this reason, the communication interface 50 may be connected to the external device 60 or the like.

As illustrated in FIG. 2, in an embodiment, the electronic device 1 may be connected to the external device 60 in a wired or wireless manner via the communication interface 50. In an embodiment, the external device 60 may be configured to include a computer of any kind, a control device of any kind, and/or the like. In an embodiment, the electronic device 1 may be configured to include the external device 60. The external device 60 may be configured in a variety of ways according to the manner in which information on heartbeat and/or heart sound detected by the electronic device 1 is to be used. Accordingly, a more detailed description of the external device 60 is omitted.

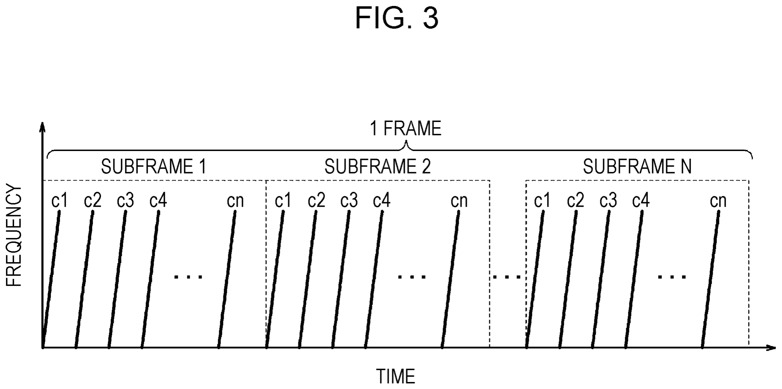

FIG. 3 is a diagram for describing an example of chirp signals generated by the signal generation processing unit 11 of the signal processing unit 10.

FIG. 3 illustrates the temporal structure of one frame in the case of using the fast-chirp modulation (FCM) scheme. FIG. 3 illustrates an example of a received signal of the FCM scheme. FCM is a scheme in which chirp signals illustrated as $c_1, c_2, c_3, c_4, ..., c_n$ in FIG. 3 are repeated at relatively short intervals (for example, equal to or greater than the round-trip time of electromagnetic waves between the radar and the target as calculated from the maximum ranging distance). In FCM, the transmission and reception processing is often divided into subframe units as illustrated in FIG. 3 for convenient signal processing of a received signal.

In FIG. 3, the horizontal axis represents elapsed time, and the vertical axis represents frequency. In the example illustrated in FIG. 3, the signal generation processing unit 11 generates linear chirp signals whose frequency varies periodically and linearly. In FIG. 3, the chirp signals are indicated as $c_1, c_2, c_3, c_4, ..., c_n$. As illustrated in FIG. 3, in each of the chirp signals, the frequency increases linearly over time.

In the example illustrated in FIG. 3, several chirp signals such as $c_1, c_2, c_3, c_4, ..., c_n$ are included as a single subframe. That is, subframe 1, subframe 2, and so on illustrated in FIG. 3 are each configured to include several chirp signals such as $c_1, c_2, c_3, c_4, ..., c_n$. In the example illustrated in FIG. 3, several subframes such as subframe 1, subframe 2, ..., subframe N are included as a single frame (1 frame). That is, the 1 frame illustrated in FIG. 3 is configured to include N subframes. The 1 frame illustrated in FIG. 3 may be frame 1, followed by frame 2, frame 3, and so on. These frames are each configured to include N subframes, in the same and/or similar manner as frame 1. A frame interval of given length may also be included between frames. The single frame illustrated in FIG. 3 may be around 30-50 milliseconds long, for example.

In the electronic device 1 according to an embodiment, the signal generation processing unit 11 may generate a transmission signal as any number of frames. In FIG. 3, some of the chirp signals are omitted from illustration. The relationship between the time and frequency of a transmission signal generated by the signal generation processing unit 11 in this way may be stored as various setting parameters in a storage unit or the like of the signal processing unit 10.

In this way, in an embodiment, the electronic device 1 may transmit a transmission signal formed from subframes including a plurality of chirp signals. In an embodiment, the electronic device 1 may transmit a transmission signal formed from frames including a given number of subframes.

The following describes the electronic device 1 as transmitting a transmission signal with a frame structure as illustrated in FIG. 3. However, the frame structure as illustrated in FIG. 3 is an example, and the chirp signals included in one subframes may be of any kind, for example. That is, in an embodiment, the signal generation processing unit 11 may generate subframes including any number of (for example, any plurality of) chirp signals. The subframe structure illustrated in FIG. 3 is also an example, and the subframes included in one frame may be of any kind, for example. That is, in an embodiment, the signal generation processing unit 11 may generate frames including any number of (for example, any plurality of) subframes. The signal generation processing unit 11 may generate signals of different frequency. The signal generation processing unit 11 may generate a plurality of discrete signals each having a different frequency f.

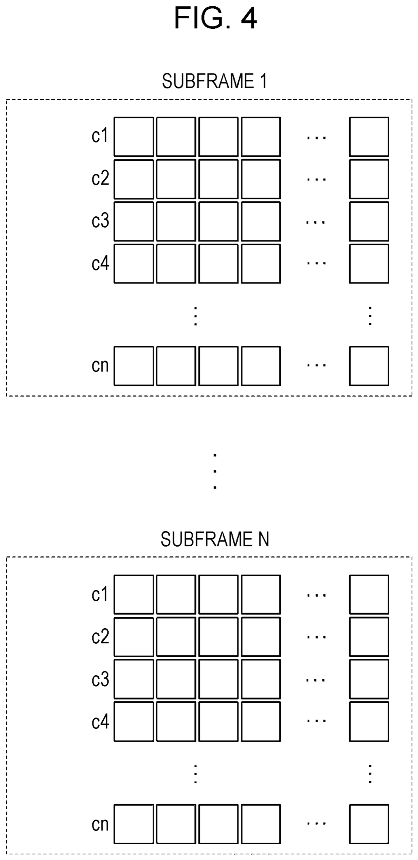

FIG. 4 illustrates another form of a portion of the subframes illustrated in FIG. 3. FIG. 4 is an illustration the result of the received signal processing unit 12 (FIG. 2) of the signal processing unit 10 performing two-dimensional fast Fourier transform (2D-FFT) processing on samples of a received signal obtained by receiving the transmission signal illustrated in FIG. 3.

As illustrated in FIG. 4, each of chirp signals $c_1, c_2, c_3, c_4, ..., c_n$ is stored in each of subframes such as subframe 1, ..., subframe N. In FIG. 4, each of the chirp signals $c_1, c_2, c_3, c_4, ..., c_n$ is formed from samples, which are indicated by the horizontally arrayed grid squares. The received signal illustrated in FIG. 4 is subjected to 2D-FFT, CFAR, integration signal processing on each subframe, and/or the like by the received signal processing unit 12 illustrated in FIG. 2.

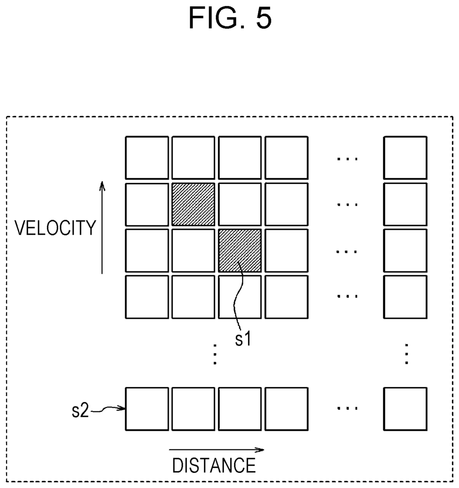

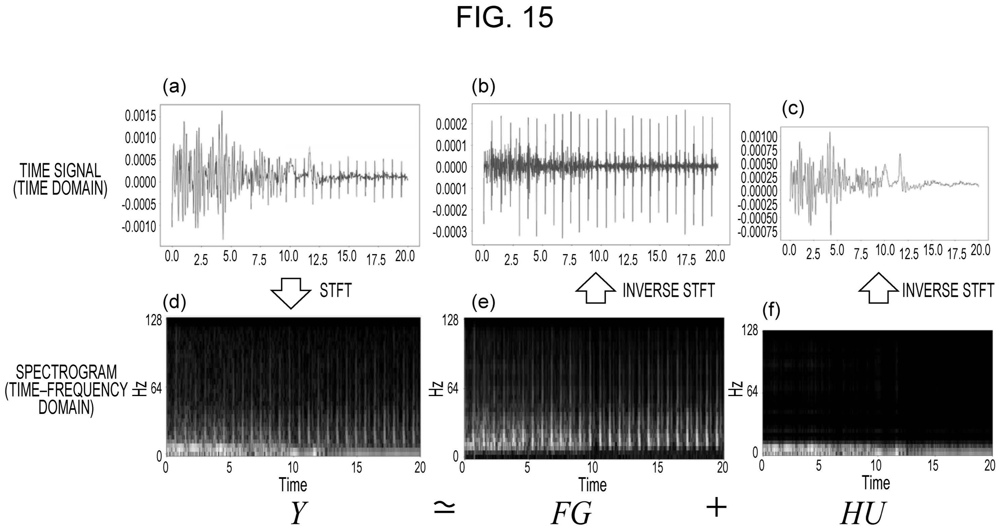

FIG. 5 illustrates an example of a point group in the range-Doppler (distancevelocity) plane calculated as a result of performing 2D-FFT, CFAR, and integration signal processing on each subframe in the received signal processing unit 12 illustrated in FIG. 2.

In FIG. 5, the horizontal direction represents range (distance), and the vertical direction represents velocity. The shaded grid square s1 illustrated in FIG. 5 illustrates a point group indicating a signal exceeding CFAR threshold processing. The non-shaded grid square s2 illustrated in FIG. 5 illustrates a bin (2D-FFT sample) with no point group, which did not exceed the CFAR threshold. Direction estimation is used to calculate the bearing, from the radar, of the calculated point group in the range-Doppler plane illustrated in FIG. 5, and the position and velocity in the two-dimensional plane are calculated as a point group indicating an object such as the test subject 200. Direction estimation may be calculated by a beamformer and/or a subspace method. Typical subspace method algorithms include MUltiple SIgnal Classification (MUSIC) and estimation of signal parameters via rotational invariant techniques (ESPRIT).

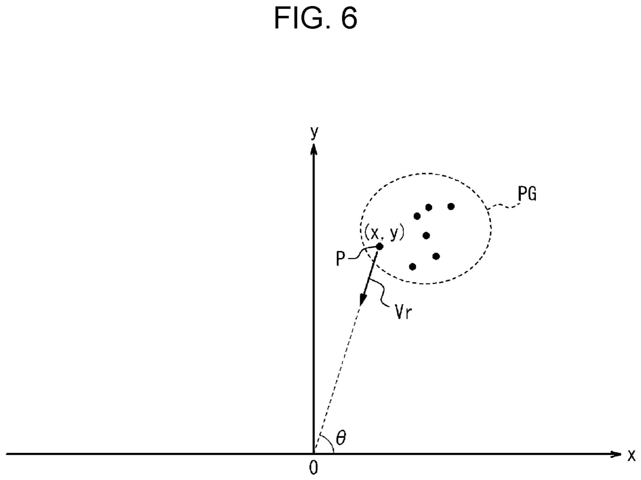

FIG. 6 illustrates an example of the result of the transformation of point group coordinates from the range-Doppler plane illustrated in FIG. 5 to the XY plane by the received signal processing unit 12 after performing direction estimation. The XY plane illustrated in FIG. 6 may be the same as the XY plane illustrated in FIG. 1. As illustrated in FIG. 6, the received signal processing unit 12 can plot a point group PG in the XY plane. The point group PG contains points P. Each of the points P has an angle $\theta$ and a radial velocity Vr in polar coordinates.

The received signal processing unit 12 detects an object present in the range where a transmission wave was transmitted, on the basis of at least one of the 2D-FFT or angle estimation results. The received signal processing unit 12 may perform object detection by performing, for example, clustering processing on the basis of respectively estimated information on distance, information on velocity, and angle information. Known algorithms used in clustering data include density-based spatial clustering of applications with noise (DBSCAN), for example. DBSCAN is an algorithm that performs density-based clustering. In the clustering processing, the average power of the points making up a detected object may be calculated, for example. The information on distance, information on velocity, angle information, and information on power pertaining to an object detected in the received signal processing unit 12 may be supplied to the external device 60 or the like via the communication interface 50, for example.

As above, the electronic device 1 may be provided with a transmitting antenna (transmitting antenna 24), a receiving antenna (receiving antenna 31), and the signal processing unit 10. The transmitting antenna 24 transmits a transmission wave formed from a radio wave, for example. The receiving antenna 31 receives a reflected wave resulting from the transmission wave being reflected. The signal processing unit 10 detects an object (an object such as the test subject 200, for example) that reflects the transmission wave, on the basis of the transmission signal transmitted as the transmission wave and the received signal received as the reflected wave.

The following further describes direction estimation of an arriving wave (an arriving reflected wave) by an antenna array of the electronic device 1 according to an embodiment.

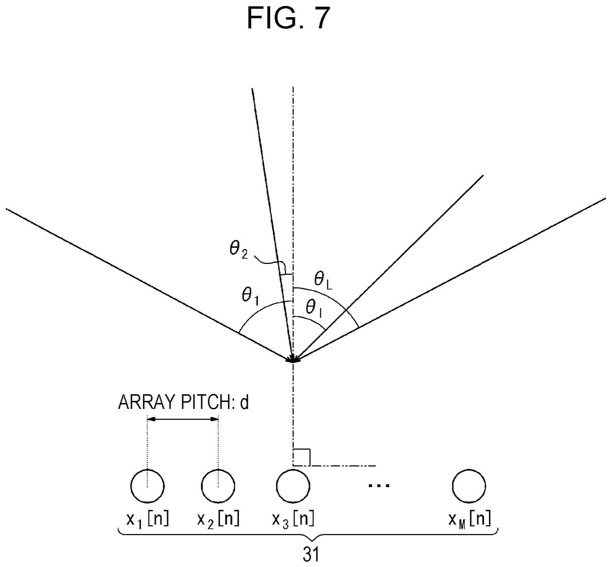

FIG. 7 is a diagram for describing the configuration of the receiving antenna 31 and the principle of direction estimation of an arriving wave by the receiving antenna 31 of the electronic device 1 according to an embodiment. FIG. 7 illustrates an example of the reception of radio waves by the receiving antenna 31.

As illustrated in FIG. 7, the receiving antenna 31 may be a linear arrangement of sensors such as receiving antennas. As illustrated in FIG. 7, in an embodiment, the receiving antenna 31 may be configured to include a plurality of receiving antennas in a linear array. In FIG. 7, the plurality of antennas $x_1, x_2, x_3, ..., x_M$ that make up the receiving antenna 31 are illustrated by small circles. The receiving antenna 31 may include any plurality of antennas. As illustrated in FIG. 7, the plurality of antennas that make up the receiving antenna 31 are spaced apart from one another by an array pitch d. This type of sensor array, in which sensors (such as antennas, ultrasonic transducers, and microphones) corresponding to various physical waves are disposed in an array, is also referred to as a uniform linear array (ULA). As illustrated in FIG. 7, the physical waves (such as electromagnetic waves and sonic waves) arrive from various directions, such as $\theta_1$ and $\theta_2$, for example. Herein, $\theta_1$ and $\theta_2$ may refer to the angle of arrival described above. In this way, a sensor array like the receiving antenna 31 can estimate the direction of arrival (angle of arrival) by utilizing the phase difference that occurs in measurement values between sensors according to the direction of arrival of a physical wave. This type of technique for estimating the direction of arrival of a wave is also referred to as angle-of-arrival estimation or direction-of-arrival (DoA) estimation.

In the electronic device 1 according to an embodiment, at least one of the transmitting antenna 24 or the receiving antenna 31 may have a plurality of antennas in a linear arrangement. This allows for appropriate narrowing of directivity in the transmission and reception of radio waves in a millimeter-wave radar, for example. When transmitting a transmission wave, the direction of the transmission beam is often controlled by a beamformer. On the other hand, when receiving a reflected wave, the direction of arrival of the reflected wave is more often estimated by subspace methods (such as MUSIC and ESPRIT described above) than by a beamformer. With beamformers and subspace methods, for electromagnetic waves arriving from various directions in the ULA as illustrated in FIG. 7, a phase difference occurs in measurement values between sensors depending on the direction of arrival. Accordingly, this phase difference can be utilized to estimate the direction of arrival of a reflected wave.

The following further describes angle estimation in two directions of an arriving wave by an antenna array of the electronic device 1 according to an embodiment.

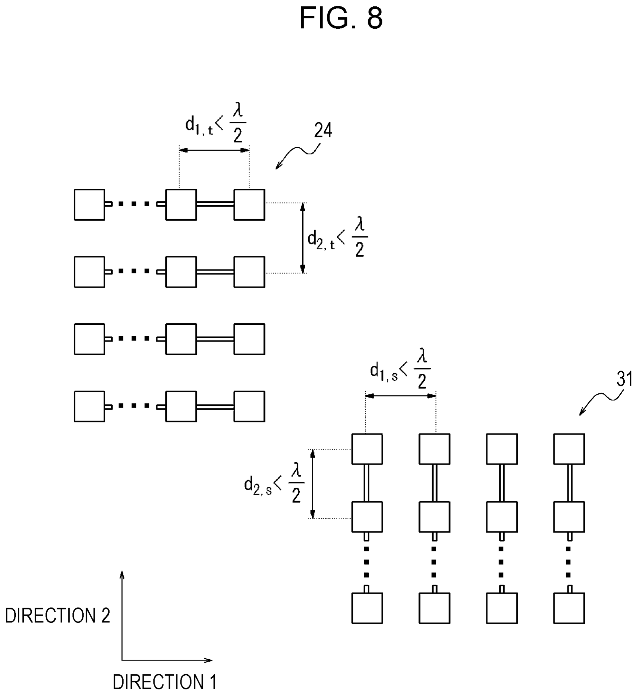

FIG. 8 illustrates an example arrangement of antennas for estimating the direction of arrival with respect to two orthogonal angles.

As illustrated in FIG. 8, in the electronic device 1 according to an embodiment, the transmitting antenna 24 and/or receiving antenna 31 may be configured to include an array of a plurality of patch antenna units.

In the transmitting antenna 24 illustrated in FIG. 8, one patch antenna unit may be configured to include a plurality of elements electrically connected in the direction of direction 1 illustrated in the diagram. In each of the patch antenna units, the plurality of elements may be electrically connected by wiring such as striplines on a board, for example. In each of the patch antenna units, the plurality of elements may be spaced apart from one another by a pitch $d_{1,1}$ shorter than half the wavelength $\lambda$ of the transmission wave. In FIG. 8, each of the patch antenna units may have any number of two or more elements electrically connected.

As illustrated in FIG. 8, the transmitting antenna 24 may include a plurality of patch antenna units arrayed in the direction of direction 2 illustrated in the diagram. The patch antenna units may be spaced apart from one another by a pitch $d_{2,t}$ shorter than half the wavelength $\lambda$ of the transmission wave. In an embodiment, the transmitting antenna 24 may include any number of two or more patch antenna units.

As illustrated in FIG. 8, in an embodiment, the receiving antenna 31 may be a variation of the arrangement of the plurality of elements in the transmitting antenna 24. That is, in the receiving antenna 31 illustrated in FIG. 8, one patch antenna unit may be configured to include a plurality of elements electrically connected in the direction of direction 2 illustrated in the diagram. In each of the patch antenna units, the plurality of elements may be electrically connected by wiring such as striplines on a board, for example. In each of the patch antenna units, the plurality of elements may be spaced apart from one another by a pitch $d_{2,s}$ shorter than half the wavelength $\lambda$ of the transmission wave. In FIG. 8, each of the patch antenna units may have any number of two or more elements electrically connected.

As illustrated in FIG. 8, the receiving antenna 31 may include a plurality of patch antenna units arrayed in the direction of direction 1 illustrated in the diagram. The patch antenna units may be spaced apart from one another by a pitch $d_{1,s}$ shorter than half the wavelength $\lambda$ of the transmission wave. In an embodiment, the receiving antenna 31 may include any number of two or more patch antenna units.

The elements included in the transmitting antenna 24 and the receiving antenna 31 may all be arranged in the same plane (on the surface layer of the same board, for example). The transmitting antenna 24 and the receiving antenna 31 may also be arranged in proximity to each other (monostatic). Direction 1 and direction 2 illustrated in FIG. 8 may be geometrically orthogonal.

With the transmitting antenna 24 and the receiving antenna 31 as illustrated in FIG. 8, the directivity of each of the transmitting antenna and the receiving antenna can be narrowed appropriately. By using the transmitting antenna 24 as illustrated in FIG. 8 to control the direction in which to transmit each transmission wave at each timing for transmitting a transmission wave (transmission signal), a beamformer can be realized for the direction of direction 2 illustrated in FIG. 8. By using the receiving antenna 31 as illustrated in FIG. 8, estimation of the direction of arrival of the reflected wave can be realized for the direction of direction 1 illustrated in FIG. 8. This enables estimation of the direction of arrival of a reflected wave with respect to two substantially orthogonal angles. Accordingly, a point group indicating an object such as the test subject 200 can be acquired three-dimensionally.

The following describes a technique for detecting the heartbeat of the test subject 200 by using the electronic device 1 according to an embodiment.

In an embodiment, the electronic device 1 measures (estimates) the heartbeat of the test subject 200 on the basis of the result of transmitting a millimeter-wave radar or other transmission wave toward the test subject 200 and receiving a reflected wave that was reflected in the chest area of the test subject 200 where the heart resides. As described above, the test subject 200 may be a human being, and may also be an animal. In this case, for example, the vibrations at the position where the test subject 200 is detected to be present by the radar can be filtered by frequency to extract components assumed to be the envelope curve of the heartbeat. Once the components assumed to be the envelope curve of the heartbeat are extracted, the peak-to-peak intervals of the envelope curve can be obtained as the heartbeat interval to roughly calculate the heartbeat interval. The approximation that peaks of the heartbeat envelope roughly correspond to R peaks in an electrocardiogram can be used. Therefore, the "heartbeat interval" may also be referred to as the RR interval (or RRI), like the terms used for electrocardiograms and the like.

The following is an examination of techniques for estimating the heartbeat interval of the test subject 200 from the result of performing the 2D-FFT, CFAR processing, and direction-of-arrival estimation described above and the like. The following describes the manner in which the heartbeat or body motion of a person is expressed as a result of the 2D-FFT performed in FIGs. 4 and 5.

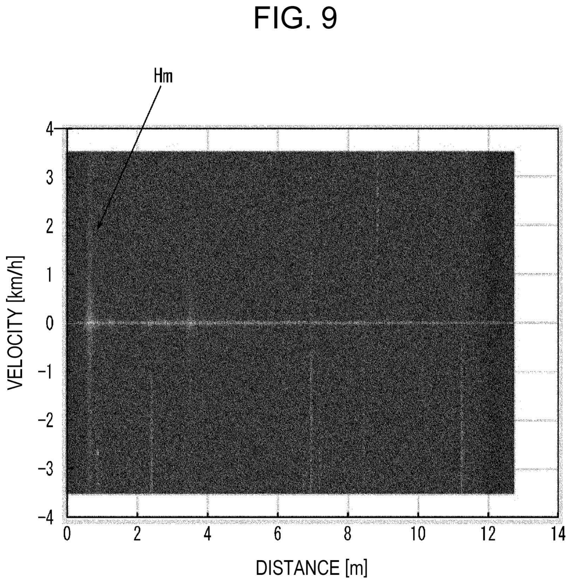

FIG. 9 illustrates an example of the result of receiving and performing 2D-FFT processing on reflected wave of a transmission wave transmitted toward the test subject 200. FIG. 9 illustrates a spectrum illustrating the heart sound and body motion of the test subject 200 as a result of the 2D-FFT. In FIG. 9, the horizontal axis represents distance (range), and the vertical axis represents velocity. In the electronic device 1 according to an embodiment, the signal processing unit 10 (for example, the heartbeat extraction unit 13) may extract a peak Hm as illustrated in FIG. 9, for example, as being body motion such as the heartbeat of the test subject 200. The spectral component indicated by the peak Hm in FIG. 9 includes not only the heart sound of the test subject 200 and the envelope curve of the heart sound, but also body motion. To extract the heartbeat interval, it may be necessary to extract body motion and the like, and thus frequency filtering is assumed to be performed, for example. The frequency filtering performed at this point is assumed to be, for example, a bandpass filter, high-pass filter, and/or low-pass filter targeting frequencies between 0.5 Hz and 10 Hz inclusive.

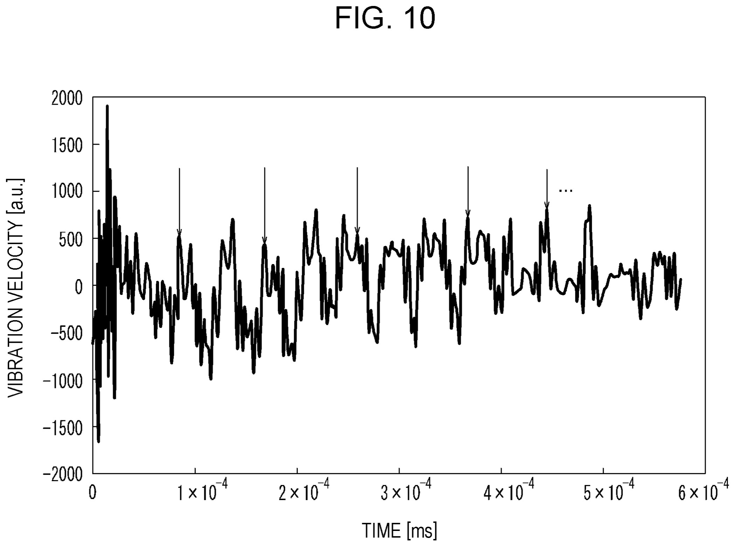

FIG. 10 is a diagram for describing a method of detecting peaks on the basis of the envelope waveform of the heart sound obtained by the frequency filtering described above. The graph illustrated in FIG. 10 illustrates an example of temporal changes in the heart sound envelope waveform of the heart sound extracted by the frequency filtering described above. A heart sound waveform as illustrated in FIG. 10 can be obtained by the technique of reconstructing a time waveform from the result of performing frequency analysis, such as the inverse Fourier transform, on the 2D-FFT data illustrated in FIG. 9. The envelope waveform illustrated in FIG. 10 includes many peaks as illustrated in the diagram. On the other hand, the beating of the test subject 200 is known to fall within the range approximately from 50 to 130 beats per minute. Therefore, the rough heart sound interval of the test subject 200 can be calculated by selecting, from among the many peaks illustrated in FIG. 10, the peaks having a time interval from 0.4 seconds to 0.8 seconds, that is, the inverse of the number of beats per minute. For example, the peaks indicated by the downward-pointing arrows in FIG. 10 may be selected as the rough heart sound interval of the test subject 200. The heart rate of the test subject 200 can be calculated from the interval of heartbeats as seen in a section containing several peaks indicated by the downward-pointing arrows in FIG. 10.

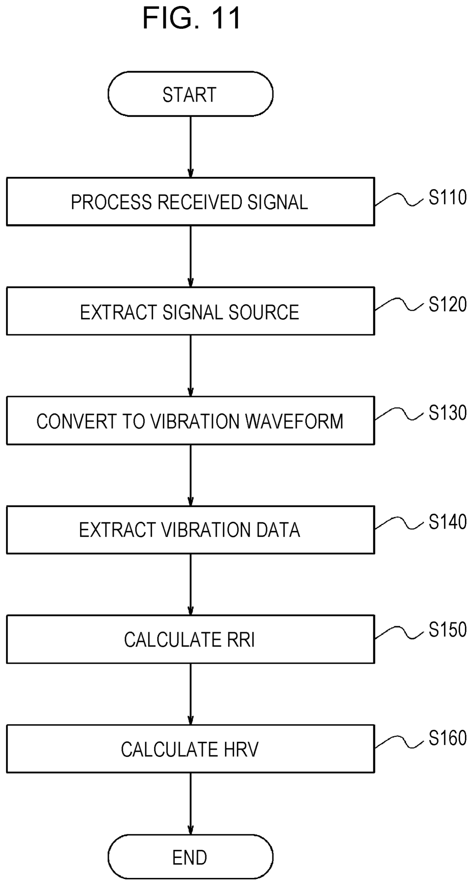

FIG. 11 is a flowchart illustrating an example of the heartbeat interval estimation operations described above. The following refers to FIG. 11 to summarize the heartbeat interval estimation operations described above.

FIG. 11 illustrates operations after the electronic device 1 according to an embodiment has received a reflected wave. That is, the operations illustrated in FIG. 11 presuppose that the electronic device 1 illustrated in FIG. 2 is transmitting a transmission wave (transmission signal) from the transmitting antenna 24. At least a portion of the transmission wave transmitted from the electronic device 1 is reflected by the test subject 200 (the chest portion thereof, for example) to become a reflected wave. The electronic device 1 illustrated in FIG. 2 receives such a reflected wave from the receiving antenna 31 and converts the reflected wave into a digital signal (reception ADC 34). From that point, the operations illustrated in FIG. 11 begin.

When the operations illustrated in FIG. 11 begin, in step S110, the signal processing unit 10 of the electronic device 1 processes the signal that is received (received signal). The signal processing performed in step S110 may include the 2D-FFT, CFAR processing, and/or direction-of-arrival estimation described above, for example. Such operations may be performed by the received signal processing unit 12 of the signal processing unit 10, for example.

In step S120, the signal processing unit 10 extracts a signal source from the information processed in step S110. The signal processing performed in step S120 may include processing for filtering the data that is the result of performing the 2D-FFT processing, for example. In step S120, the signal processing unit 10 may extract a spectral component at only the position where the test subject 200 is present. The position where the test subject 200 is present may be identified by any of various known techniques. Such operations may be performed by the heartbeat extraction unit 13 of the signal processing unit 10, for example.

In step S130, the signal processing unit 10 converts the processing result of the previous stage into a vibration waveform. The processing performed in step S130 may include processing to extract phase information from IQ data, for example. In step S130, the signal processing unit 10 may include processing to extract vibration data including heart sound from the spectral component of the 2D-FFT processing of the test subject 200 extracted in step S120. Such operations may be performed by the heartbeat extraction unit 13 of the signal processing unit 10, for example.

In step S140, the signal processing unit 10 extracts vibration data from the processing result of the previous stage. The processing performed in step S140 may include frequency filtering, for example. In step S140, the signal processing unit 10 may perform frequency filtering to extract a low-frequency signal including the envelope curve of the heart sound of the test subject 200. Such operations may be performed by the heartbeat extraction unit 13 of the signal processing unit 10, for example.

In step S150, the signal processing unit 10 calculates the RRI interval (RRI) of the test subject 200 by detecting peaks in the beating of the test subject 200 from the processing result of the previous stage and calculating the interval between the peaks. In step S150, the signal processing unit 10 may detect peaks in the low-frequency signal including the envelope curve of the heart sound of the test subject 200. In step S150, the signal processing unit 10 may also calculate and/or extract the interval between peaks. Such operations may be performed by the calculation unit 14 of the signal processing unit 10, for example. As above, in step S150, the signal processing unit 10 may extract the heart sound interval.

In step S160, the signal processing unit 10 may calculate the heart rate variability (HRV) of the test subject 200. In step S160, the signal processing unit 10 may calculate the HRV of the test subject 200 by calculating the spectral density of RRI time-series data. Calculating the power spectral density of RRI time-series data may involve using Welch's method or the like to perform frequency analysis on an RRI time-series waveform. Such operations may be performed by the calculation unit 14 of the signal processing unit 10, for example. In step S160, the signal processing unit 10 may also analyze the spectrum from the processing result of the previous stage.

As above, in an embodiment, the electronic device 1 performs frequency filtering on vibrations at the position where the test subject 200 is present to extract components thought to be the envelope curve of the heartbeat, and obtains the interval between peaks of the envelope curve as the heartbeat interval. In this way, in an embodiment, the electronic device 1 can calculate the rough heartbeat interval of the test subject 200.

For example, by being installed in a moving body such as an automobile, the electronic device 1 according to an embodiment can detect the heartbeat and the like of an occupant on board the moving body. In this case, the electronic device 1 according to an embodiment can analyze the RRI of a driver who is operating the moving body such as an automobile by performing radar-based heartbeat measurement on the driver. If such RRI analysis can be performed with relatively high precision, the electronic device 1 can be used to estimate the psychological state, including level of alertness, of an occupant such as the driver, for example. If such RRI analysis can be performed with relatively high precision, the electronic device 1 can also be used to detect distracted driving and the like, including the driver falling asleep at the wheel, or to detect predictive signs of such distracted driving.

On the other hand, when a moving body such as an automobile in which the electronic device 1 is installed travels on a road, various types of vibrations frequently occur, including road noise caused by unevenness of the road surface, for example. In such situations, vibrations other than pulsation are superimposed as noise onto the skin surface of the driver or other occupant of the moving body such as an automobile. Consequently, the precision of occupant heartbeat detection by the electronic device 1 may be affected while the moving body is in motion.

Accordingly, in an embodiment, the electronic device 1 further improves on the technique described above. According to an embodiment, the electronic device 1 extracts heart sound by radar using a high-frequency band at or above millimeter waves, for example, and analyzes the heart sound to achieve accurate heartbeat interval extraction. According to an embodiment, the electronic device 1 achieves highly precise waveform extraction, even for a heart sound waveform with superimposed noise such as road noise, for example.

According to an embodiment, the electronic device 1 executes signal processing, including the Fourier transform, in an appropriate sequence on the basis of a transmission wave and a reflected wave from a millimeter-wave radar, for example. According to an embodiment, the electronic device 1 narrows down the subspace and the subspace basis containing the heart sound from the reflected wave and selects the most plausible heart sound from the obtained waveform. According to an embodiment, the electronic device 1 removes noise. In this way, according to an embodiment, the electronic device 1 achieves heart sound waveform acquisition. According to an embodiment, the electronic device 1 can calculate the heartbeat interval by applying signal processing in an appropriate sequence.

The following describes a technique like the above. In an embodiment, to extract the heart sound of the test subject 200 accurately, the electronic device 1 performs appropriate signal processing on signals (chirp signals) received by the electronic device 1 in an appropriate sequence. In this way, in an embodiment, the electronic device 1 narrows down the subspace and the subspace basis in which the signal components of the heart sound of the subject 200 exist. In an embodiment, the electronic device 1 represents the time-series signal initially in the form of a linear combination made up of multiple subspace bases. From the result, the electronic device 1 extracts subspaces that have a large contribution ratio for representing the heart sound. The electronic device 1 uses only the extracted subspaces to construct the time-series signal again. In an embodiment, the electronic device 1 can use such processing to search for subspaces in which the heart sound of the test subject 200 exists. To achieve such a technique, in an embodiment, the electronic device 1 may execute processing that involves subjecting a reflected wave from a radar to 2D-FFT processing, other Fourier transforms, wavelet transform, or a derivative thereof, for example.

In an embodiment, the electronic device 1 may execute characteristic processing like the following on a received signal according to a step-by-step procedure. The following summarizes characteristic processing by the electronic device 1 according to an embodiment. In an embodiment, the electronic device 1 may execute the following two characteristic processes. Namely, in an embodiment, the electronic device 1 executes (1) Subspace extraction processing and (2) Statistical signal processing for noise removal. The following describes each of these processes in greater detail.

(1) Subspace extraction processing

In an embodiment, the electronic device 1 executes processing on a received signal to omit data considered unnecessary in a linear space. In an embodiment, the electronic device 1 may execute processing to omit unnecessary subspaces, thereby leaving the necessary subspaces. Specifically, processing like the following may be executed.

(1-1)

The electronic device 1 applies two Fourier transforms to a received chirp signal. The first Fourier transform can be used to obtain the distance between the electronic device 1 and the object that reflected the transmission wave. The second Fourier transform can be used to obtain the velocity, relative to the electronic device 1, of the object that reflected the transmission wave. The electronic device 1 may implement a window function, as appropriate, to maintain the periodicity of the signal used for the Fourier transforms. The electronic device 1 may extract only a micro-Doppler component that includes a point group where the test subject 200 (for example, a human or an animal) is present by estimating the direction of arrival of the reflected wave.

(1-2)

The electronic device 1 executes, on the micro-Doppler signal, processing to select the signal corresponding to the vibration of the body surface from out of the set of multiple time-series signals (based on vibration) extracted in the processing in the above section (1-1). The electronic device 1 executes, on the selected signal, processing based on the inverse Fourier transform, an inverse wavelet transform, or a derivative thereof to revert the selected signal back to a time signal.

(1-3)

The electronic device 1 extracts, from the time signal obtained by the processing in the above section (1-2), only subspaces constituting heart sound according to one of the following techniques: processing based on the short-time Fourier transform, a wavelet transform, or a derivative thereof; or a bandpass filter. The electronic device 1 reconstructs the time signal from the components of the extracted subspaces (constituting heart sound). At this point, the electronic device 1 can obtain a waveform representing heart sound.

(2) Statistical signal processing for noise removal

In an embodiment, to remove noise superimposed on the data of the detected heart sound, the electronic device 1 may execute processing like the following.

(2-1)

If the electronic device 1 is installed in a moving body such as an automobile, for example, there is a strong possibility that body motion of an occupant, vibrations during travel, and/or the like are superimposed on the heart sound waveform obtained by the processing in the above section (1-3). Accordingly, the electronic device 1 generates a spectrogram or a scalogram by subjecting the heart sound waveform obtained by the processing in the above section (1-3) to frequency analysis using any of the short-time Fourier transform or a wavelet transform. In the following, the spectrogram or the scalogram may be referred to collectively as "time-frequency analysis information", as appropriate.

(2-2)

The electronic device 1 uses semi-supervised non-negative matrix factorization to approximate the time-frequency analysis information generated by the processing in the above section (2-1) as the sum of two matrix products (each being the product of a frequency basis matrix and an activation matrix). In the following, semi-supervised non-negative matrix factorization may be referred to as SSNMF, as appropriate. One of the two frequency basis matrices is obtained in advance from a resting human heart sound waveform. In the remaining three matrices, each matrix element may be assumed to be updated by an update formula for minimizing a cost function determined by the non-negative matrix factorization. The updating of the matrix elements may be repeated a predetermined number of iterations, or until the cost function falls below a predetermined threshold. In the non-negative matrix factorization, it may be necessary to stipulate the number of basis vectors of the frequency basis matrix. Accordingly, the electronic device 1 may set the number of basis vectors of the frequency basis matrix to more than one number and perform processing based on SSNMF for each set number of bases.

(2-3)

The electronic device 1 calculates the time-frequency analysis information, including a subspace representing the heartbeat, from the matrix product of the frequency basis matrix obtained in advance and a corresponding activation matrix (obtained by the processing in the above section (2-2)). The electronic device 1 combines the calculated time-frequency analysis information with phase information about the time-frequency analysis information generated by the processing in the above section (2-1). Such processing allows the electronic device 1 to obtain a heart sound waveform with the noise component removed.

(2-4)

The electronic device 1 evaluates the presence or absence of noise in the waveform obtained by the processing in the above section (2-3) by calculating a degree of abnormality using singular spectrum transformation (SST) for each variety of the set number of bases. The heart sound waveform is basically a periodic signal as long as the waveform does not contain components other than heart sound. Under normal conditions, it is difficult to imagine that rapid fluctuations would occur in the heartbeat interval over a short time. Accordingly, there is a strong possibility that an increase in the degree of abnormality according to SST means that noise is superimposed. On the basis of such a principle, the electronic device 1 may obtain a representative indicator such as the maximum, the median, or a weighted average of the degrees of abnormality obtained from waveforms corresponding to respective varieties of the number of bases, and the number of bases for which the representative indicator is minimized may be used as the best parameter. The electronic device 1 may use this parameter in the calculation of the heartbeat interval, which is the processing to be performed next on the obtained heart sound waveform.

The following describes in greater detail operations by the electronic device 1 according to an embodiment.

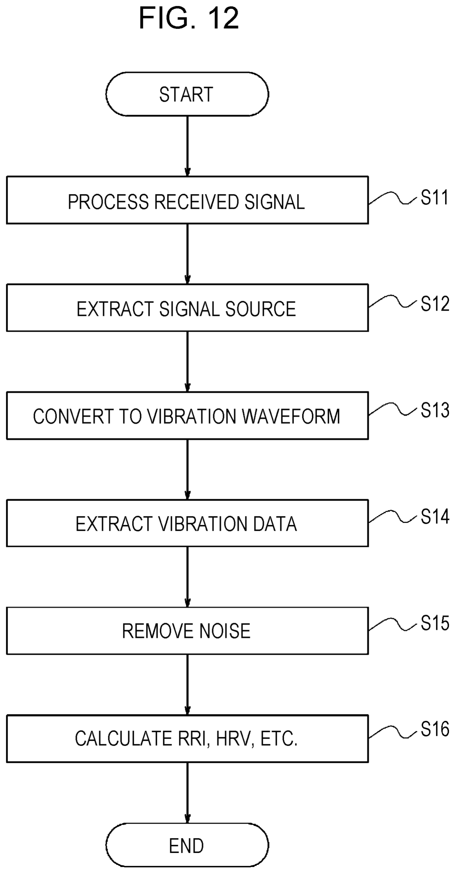

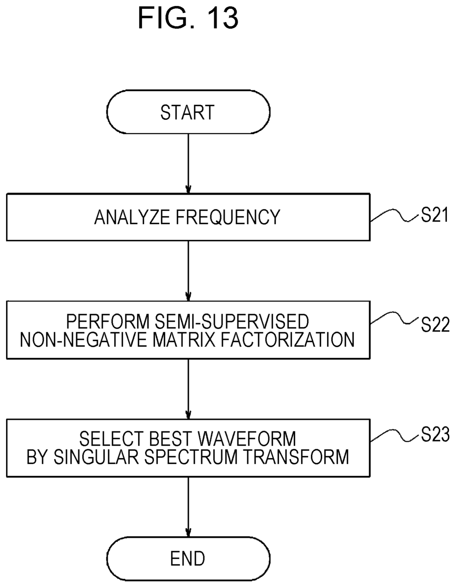

FIG. 12 is a flowchart illustrating an example of operations by the electronic device 1 according to an embodiment. FIG. 13 is a flowchart illustrating in greater detail an example of the operations in step S16 of FIG. 12. The following refers to FIGs. 12 and 13 to describe the flow of operations performed by the electronic device 1 according to an embodiment.

Steps S11 to S14 illustrated in FIG. 12 can be performed in the same and/or similar manner to the operations in steps S110 to S140 illustrated in FIG. 11. Step S16 illustrated in FIG. 12 can be performed in the same and/or similar manner to the operations in steps S150 and S160 illustrated in FIG. 11. Accordingly, a more detailed description of these operations is omitted. In an embodiment, the electronic device 1 may subject the vibration data extracted in step S14 illustrated in FIG. 12 to noise removal (step S15), and then use the data with noise removed as a basis for calculating the RRI, HRV, and the like (step S16).

The above section "(1) Subspace extraction processing" summarizes the processing in steps S11 to S14 illustrated in FIG. 12.

The processing in step S15 illustrated in FIG. 12 may be performed assuming an environment in which vibration occurring during the travel of a moving body, such as an automobile for example, in which the electronic device 1 is installed, body motion, excluding heartbeat and respiration, of an occupant, and/or the like are generated as noise. In such cases, by removing noise as described above (step S15) before performing the subsequent processing to calculate the heartbeat interval (step S16), the electronic device 1 can detect the heartbeat of an occupant with good precision.

In step S15, the signal processing unit 10 of the electronic device 1 may execute SSNMF-based iterative processing and SST-based hyperparameter tuning. More specifically, in step S15, the signal processing unit 10 may execute the processing in steps S21 to S23 illustrated in FIG. 13. The following references FIG. 13 to further describe the processing in steps S21 to S23.

Upon executing the processing in step S14 of FIG. 12, the electronic device 1 can acquire a heartbeat waveform in which pulsations corresponding to R waves (upward narrow waves) are seen to occur at fixed intervals. However, as described above, since noise may be superimposed depending on the environment in which the electronic device 1 performs detection, such noise may possibly affect the RRI calculation precision. Accordingly, the electronic device 1 removes noise superimposed on the heartbeat waveform by executing the processing illustrated in step S15 of FIG. 12 (the processing illustrated in steps S21 to S23 of FIG. 13).

When the operations illustrated in FIG. 13 start, the signal processing unit 10 of the electronic device 1 first performs frequency analysis (step S21). In step S21, the signal processing unit 10 may perform frequency analysis on the heart sound waveform to convert the heart sound waveform into time-frequency analysis information. As described above, in an environment in which the heartbeat of an occupant is detected by the electronic device 1 installed inside a moving body such as an automobile that is in motion, noise may be superimposed, and therefore there is a strong possibility that vibrations due to body motion or travel are superimposed on the detected heart sound waveform. Accordingly, in step S21, the signal processing unit 10 performs frequency analysis using any of the short-time Fourier transform or a wavelet transform on the detected heart sound waveform to generate (calculate) a spectrogram or a scalogram (time-frequency analysis information).

For example, the signal processing unit 10 may calculate a spectrogram according to the following expressions (1) and (2).

[Math. 1]

$$Y(\omega, m) = \sum_{n=0}^{N-1} x_m(n) e^{-j2\pi\omega n/N} \qquad (1)$$

[Math. 2]

$$x_m(t - mR) = g(t - mR)x(t) \qquad (2)$$

In expression (1) and/or expression (2) above, Y represents the spectrogram, x(t) represents a finite-length time-series signal (heart sound waveform), and g(t) represents a window function. Also, t (where $t \geq 0$) indicates a time variable, N indicates the frame of the Fourier transform, R indicates the hop size, and $\omega$ indicates an index of discrete frequency. Also, m indicates a variable indicating the position of a time section in which the Fourier transform is performed, the numerical value of which is assumed to fall in the range [0, T-1]. Also, a time variable such that n = t - mR is newly defined. Also, the variable n varies in the range from 0 to the data length of the Fourier transform ($0 \leq n \leq N-1$). Also, $x_m$ represents the time-series signal with the m-th index of the time frame onto which the window function is applied. The hop size R is equal to the difference between the length of the Fourier transform window and the overlap length between windows.

The spectrogram Y that is ultimately generated (calculated) is expressed as in the following expression (3), and has a size in which the number of rows is equal to the number $\Omega$ (equal to N/2) of frequency indices (bins) and the number of columns is equal to the number T of frames in the time direction.
[Math. 3]

$$Y\left(\in \mathbb{R}_{\geq 0}^{\Omega \times T}\right) \quad (3)$$

After step S21, the signal processing unit 10 executes semi-supervised non-negative matrix factorization (SSNMF) (step S22). In step S22, the signal processing unit 10 may approximate the time-frequency analysis information (for example, the spectrogram Y) obtained in step S21 as two matrix products according to an update formula. That is, the signal processing unit 10 uses SSNMF to approximate the time-frequency analysis information calculated in step S21 as the sum of two matrix products (each being the product of a frequency basis matrix and an activation matrix).
[Math. 4]

$$Y \simeq FG + HU \quad (4)$$

In expression (4) above, the matrix Y represents the time-frequency analysis information calculated in step S21 (for example, a spectrogram expressed as in expression (3) above). The matrix F represents a frequency basis matrix with a number K of bases, obtained in advance from a resting human heart sound waveform. The matrix F is expressed as in the following expression (5).
[Math. 5]

$$F\left(\in \mathbb{R}_{\geq 0}^{\Omega \times K}\right) \quad (5)$$

The matrix G represents an activation matrix corresponding to the matrix F. The matrix G is expressed as in the following expression (6).
[Math. 6]

$$G\left(\in \mathbb{R}_{\geq 0}^{K \times T}\right) \quad (6)$$

In expression (4) above, the matrix H represents a frequency basis matrix with a number L of bases, this matrix being for constructing a waveform other than the objective signal (in other words, noise). The matrix H is expressed as in the following expression (7).
[Math. 7]

$$H\left(\in \mathbb{R}_{\geq 0}^{\Omega \times L}\right) \quad (7)$$

The matrix U represents an activation matrix corresponding to the matrix H. The matrix U is expressed as in the following expression (8).
[Math. 8]

$$U\left(\in \mathbb{R}_{\geq 0}^{L \times T}\right) \quad (8)$$

The signal processing unit 10 updates the matrix elements in each of the three matrices other than the matrix F described above (in other words, the matrix G, the matrix H, and the matrix U) according to an update formula for minimizing the cost function determined by the non-negative matrix factorization. Several candidates are possible for the cost function to be applied at this point. The following describes two cost functions, namely Euclidean distance (the Frobenius norm) and information divergence (I-divergence).
A cost function based on Euclidean distance can be expressed as in the following expression (9).
[Math. 9]

$$J_{SSNMF} = \sum_{\omega,t}\left\{y_{\omega,t} - \left(\sum_k f_{\omega,k}g_{k,t} + \sum_l h_{\omega,l}u_{l,t}\right)\right\}^2 \qquad (9)$$

In expression (9) above, $y_{\omega,t}$ represents an element of the matrix Y, $f_{\omega,k}$ represents an element of the matrix F, $g_{k,t}$ represents an element of the matrix G, and $h_{\omega,l}$ represents an element of the matrix H, $u_{l,t}$ represents an element of the matrix U. Each of these elements takes a non-negative value.

A cost function based on I-divergence can be expressed as in the following expression (10).

[Math. 10]

$$E_{SSNMF} = \sum_{\omega,t}\left\{y_{\omega,t}\log\frac{y_{\omega,t}}{\sum_k f_{\omega,k}g_{k,t}+\sum_l h_{\omega,l}u_{l,t}} - \left(y_{\omega,t} - \left(\sum_k f_{\omega,k}g_{k,t} + \sum_l h_{\omega,l}u_{l,t}\right)\right)\right\} \qquad (10)$$

A cost function like those expressed in expressions (9) and (10) above can be minimized by using the auxiliary function method.

For example, an update formula corresponding to the cost function based on Euclidean distance expressed in expression (9) above can be expressed as in the following expressions (11), (12), and (13).

[Math. 11]

$$h_{\omega,l} = \frac{\sum_t y_{\omega,t}u_{l,t}}{\sum_t u_{l,t}\sum_l h_{\omega,l}u_{l,t}+\sum_t u_{l,t}\sum_k f_{\omega,k}g_{k,t}}h_{\omega,l} \qquad (11)$$

[Math. 12]

$$u_{l,t} = \frac{\sum_\omega y_{\omega,t}h_{\omega,l}}{\sum_\omega h_{\omega,l}\sum_l h_{\omega,l}u_{l,t}+\sum_\omega h_{\omega,l}\sum_k f_{\omega,k}g_{k,t}}u_{l,t} \qquad (12)$$

[Math. 13]

$$g_{k,t} = \frac{\sum_\omega y_{\omega,t}f_{\omega,k}}{\sum_\omega f_{\omega,k}\sum_k f_{\omega,k}g_{k,t}+\sum_\omega f_{\omega,k}\sum_l h_{\omega,l}u_{l,t}}g_{k,t} \qquad (13)$$

An update formula corresponding to the cost function based on I-divergence expressed in expression (10) above can be expressed as in the following expressions (14), (15), and (16).

[Math. 14]

$$h_{\omega,l} = \frac{\sum_t y_{\omega,t}u_{l,t}\left(\sum_k f_{\omega,k}g_{k,t}+\sum_l h_{\omega,l}u_{l,t}\right)^{-1}}{\sum_t u_{l,t}}h_{\omega,l} \qquad (14)$$

[Math. 15]

$$u_{l,t} = \frac{\sum_\omega y_{\omega,t}h_{\omega,l}\left(\sum_k f_{\omega,k}g_{k,t}+\sum_l h_{\omega,l}u_{l,t}\right)^{-1}}{\sum_\omega h_{\omega,l}}u_{l,t} \qquad (15)$$

[Math. 16]

$$g_{k,t} = \frac{\sum_\omega y_{\omega,t}f_{\omega,k}\left(\sum_k f_{\omega,k}g_{k,t}+\sum_l h_{\omega,l}u_{l,t}\right)^{-1}}{\sum_\omega f_{\omega,k}}g_{k,t} \qquad (16)$$

The signal processing unit 10 may repeatedly update the matrix elements a predetermined fixed number of iterations, or until the cost function falls below a predetermined threshold. In the non-negative matrix factorization, it may be necessary to stipulate the number L of basis vectors of the frequency basis matrix. Accordingly, the signal processing unit 10 may set the number of basis vectors of the frequency basis matrix to more than one number and perform processing based on SSNMF for each set number of bases. The signal processing unit 10 uses phase information about the matrix Y to revert the matrix FG (time-frequency analysis information) obtained upon finishing the SSNMF-based processing back to a time signal according to any of the inverse short-time Fourier transform, an inverse wavelet transform, or a derivative based thereon.

For example, in the case of using the inverse short-time Fourier transform, the signal processing unit 10 may perform calculations in accordance with the following expressions (17) to (20).

[Math. 17]

$$Y' = (FG) * e^{j\angle Y} \left( \in \mathbb{R}_{\geq 0}^{\Omega \times T} \right) \quad (1\ 7)$$

[Math. 18]

$$\hat{x}(t) = \sum_{m=0}^{T-1} \hat{x}_m (t - mR) \quad (1\ 8)$$

[Math. 19]

$$= \frac{1}{N} \sum_{m=0}^{T-1} g(t - mR) \times \sum_{\omega=0}^{N-1} Y'(\omega, m) e^{\frac{j2\pi\omega(t-mR)}{N}} \ (0 \leq t \leq DataNum) \quad (1\ 9)$$

[Math. 20]

$$\hat{x}_m(n) = g(n) \frac{1}{N} \sum_{\omega=0}^{N-1} Y'(\omega, m) e^{\frac{j2\pi\omega n}{N}} \quad (2\ 0)$$

In expressions (17) to (20) above, R indicates the hop size between successive DFTs. Also, Y'($\omega$, m) represents a specific element present in the spectrogram matrix obtained by SSNMF. Although there are $\Omega$ varieties of the number of discrete frequency bins of Y', but for the inverse Fourier transform, the numbers of bins are treated as if there are N varieties, which is double $\Omega$. This is because the power spectrum of a Fourier transform performed on a real signal is a symmetrical numerical value when an intermediate frequency is used as the axis, and thus when deriving the spectrogram, information is stored from the 0th to the (N/2)th discrete frequency index.

The signal processing unit 10 may apply inverse the inverse discrete Fourier transform (DFT) to the result of the DFT obtained in each frame to acquire the time signal expressed on the left side of expression (20) above. The signal processing unit 10 may calculate a reconstructed heart sound waveform (expressed on the left side of expression (18) above) by performing overlapping addition of all of the above signals. The domain of the time variable t is assumed to be from 0 to the length of the time-series signal.

When processing as described above is applied to the matrix HU rather than the matrix FG, a waveform interpreted as noise is reconstructed.

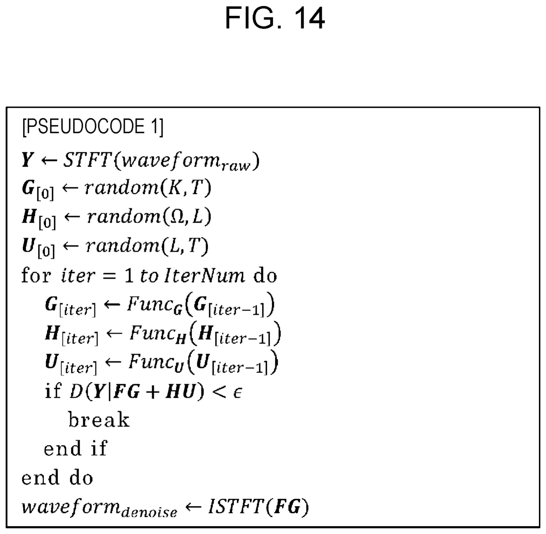

FIG. 14 illustrates an example in which a series of processing steps mostly involving the SSNMF described above is summarized as pseudocode. FIG. 15 summarizes the series of processes described above by using heart sound waveforms and spectrograms.

FIGs. 15(a), 15(b), and 15(c) illustrated in the upper row of FIG. 15 each illustrate an example of a time signal in the time domain. FIGs. 15(d), 15(e), and 15(f) illustrated in the lower row of FIG. 15 each illustrate an example of a spectrogram in the time-frequency domain.

The time signal illustrated in FIG. 15(a) illustrates the signal (original signal) before performing the processing for removing noise. The signal processing unit 10 can obtain the spectrogram Y illustrated in FIG. 15(d) by applying the short-time Fourier transform to the time signal illustrated in FIG. 15(a). The following refers to the short-time Fourier transform (also known as the short-term Fourier transform) as the STFT, as appropriate. As described above, the signal processing unit 10 approximates the spectrogram Y illustrated in FIG. 15(d) as the sum of the matrix FG illustrated in FIG. 15(e) and the matrix HU illustrated in FIG. 15(f). The signal processing unit 10 can obtain the time signal illustrated in FIG. 15(b) by applying the inverse short-time Fourier transform (inverse STFT) to the matrix FG illustrated in FIG. 15(e). The time signal illustrated in FIG. 15(b) illustrates the signal (objective signal) after performing the processing for removing noise. In this way, the signal processing unit 10 can obtain the objective signal with noise removed (FIG. 15(b)) by performing processing for converting the matrix FG illustrated in FIG. 15(e) to a time signal. On the other hand, as described above, processing for converting the matrix HU illustrated in FIG. 15(f) to a time signal can be executed to obtain a reconstructed time signal with a waveform interpreted as noise (FIG. 15(c)).

As above, the signal processing unit 10 uses the short-time Fourier transform to convert the original waveform waveform$_{raw}$ (FIG. 15(a)) to a spectrogram (FIG. 15(d)) and generate matrices (FIGs. 15(e) and 15(f)) of predetermined size that are made up of random values (non-negative values). The signal processing unit 10 takes the matrices generated in this way as the initial values of the three matrices G, H, and U. The signal processing unit 10 updates the elements of the three matrices G, H, and U according to update formulas Func$_G$, Func$_H$, and Funcu (expressions (11) to (16) above). The signal processing unit 10 repeats such update processing a prescribed number of iterations (for example, IterNum times). In this way, the signal processing unit 10 ultimately acquires three matrices G$_{[IterNum]}$, H$_{[IterNum]}$, and U$_{[IterNum]}$. In an embodiment, if a cost function D (expressions (9) and (10) above) of Y and FG+HU falls below a threshold ε partway through the repetition described above, the signal processing unit 10 may discontinue the update processing. The signal processing unit 10 uses the inverse short-time Fourier transform to revert the spectrogram (FIG. 15(e)) back to a time signal waveform$_{denoise}$ with noise removed (FIG. 15(b)).

After step S22, the signal processing unit 10 executes processing to select the best waveform according to singular spectrum transformation (SST) (step S23). In step S23, the signal processing unit 10 may use SST to calculate a degree of abnormality of the waveform and update the optimal number of bases to thereby select the best waveform. The signal processing unit 10 may tune SST-based hyperparameters on the basis of the result obtained in step S22.

In step S23, the signal processing unit 10 may evaluate the presence or absence noise by calculating the degree of abnormality according to SST for each variety of the number L of bases. As described above, the heart sound waveform is basically a periodic signal as long as the waveform does not contain components other than heart sound. Under normal conditions, it is difficult to imagine that rapid fluctuations would occur in the heartbeat interval over a short time. Accordingly, there is a strong possibility that an increase in the degree of abnormality according to SST means that noise is superimposed. SST takes two adjacent sections from the overall time-series signal, creates two matrices (referred to as the history matrix H$_1$ and the check matrix H$_2$, respectively) from each of the time-series signals, and applies a multivariate analysis technique. SST allows for quantitative evaluation of the degree of deviation between the waveforms of the two sections.

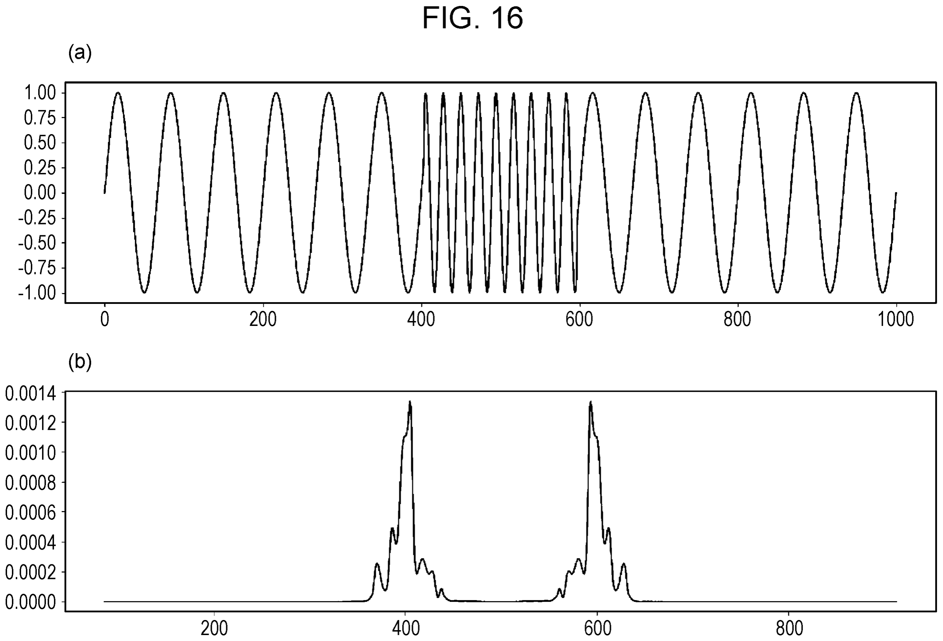

FIG. 16 illustrates an example of SST-based abnormality detection. FIG. 16(a) illustrated in the upper row of FIG. 16 represents an input waveform. FIG. 16(b) illustrated in the lower row of FIG. 16 indicates a degree of abnormality obtained through processing. As illustrated in FIG. 16(b), according to SST, the degree of abnormality can be confirmed to increase whenever the trend of the waveform changes.

The following describes processing steps based on SST. For the multivariate analysis technique, a subspace method or singular value decomposition can be used mainly. The following describes processing in the case of using singular value decomposition.

In SST, first, if the overall time-series signal is as expressed in the following expression (21) (corresponding to expression (18) above), a partial time series can be defined as in expression (22) below.

[Math. 21]

$$\{s_t \in \mathbb{R} | t = 1, 2, \dots \} \quad (2\,1)$$

[Math. 22]

$$\boldsymbol{s}(t) \equiv (s_{t-\omega+1}, \dots, s_{t-1}, s_t)^\top \in \mathbb{R}^\omega \quad (2\,2)$$

In expression (22) above, T means the transpose. The sample points of the time-series signal are assumed to be recorded at fixed time intervals. In this case, the history matrix H$_1$ and the check matrix H$_2$ containing n partial time-series signals at time t can be defined as in the following expressions (23) and (24).

[Math. 23]

$$\boldsymbol{H}_1(t) = [\boldsymbol{s}(t-n), \dots, \boldsymbol{s}(t-2), \boldsymbol{s}(t-1)] \quad (2\,3)$$

[Math. 24]

$$\boldsymbol{H}_2(t) = [\boldsymbol{s}(t-n+\gamma), \dots, \boldsymbol{s}(t-1+\gamma)] \quad (2\,4)$$

Here, $\gamma$ is an integer, the value of which dictates the distance between the two sections to be compared. The column vectors that make up these matrices $H_1$ and $H_2$ are assumed to contain pattern information that appears in the time-series data. Accordingly, the signal processing unit 10 performs singular value decomposition (SVD) on these matrices to discover characteristic patterns from the n column vectors.

The signal processing unit 10 generates matrices $U_1^{(r)}$ and $U_2^{(r)}$ containing r left singular vectors with particularly large singular values from left singular vector matrices $U_1$ and $U_2$ of the matrices $H_1$ and $H_2$ obtained through such processing. From the matrices $U_1^{(r)}$ and $U_2^{(r)}$ generated in this way, the signal processing unit 10 can calculate a degree of abnormality a(t) according to the following expressions (25) to (27).

[Math. 25]

$$H_1(t) = U_1 \Sigma_1 V_1 \qquad (2\,5)$$

[Math. 26]

$$H_2(t) = U_2 \Sigma_2 V_2 \qquad (2\,6)$$

[Math. 27]

$$a(t) = 1 - \left| U_1^{(r)^\top} U_2^{(r)} \right|_2 \qquad (2\,7)$$

The degree of abnormality a(t) defined as in expression (27) above takes a value from 0 to 1. The greater the difference between two time series, the more the degree of abnormality a(t) approaches 1. The higher the similarity between two time series, the more the degree of abnormality a(t) approaches 0. Hyperparameters to be used in this technique include the length $\omega$ of the partial time series, the number n of column vectors, the interval $\gamma$ between two sections, the number r of left singular vectors to be extracted, and the like. Depending on the combination of these hyperparameters, the sensitivity and/or stability of the abnormality detection will vary. Consequently, in SST-based abnormality detection, it may be necessary to tune the hyperparameters in advance. When SST-based abnormality detection is applied to a heart sound waveform, the hyperparameters may be set such that several heartbeat cycles are included in the partial time series. This can maintain the stability of change detection.

The signal processing unit 10 may obtain a representative indicator such as the maximum, the median, or a weighted average of the degrees of abnormality a(t) obtained from waveforms corresponding to each of the number L of bases, and a number $L_{best}$ of bases for which the representative indicator is minimized may be used as the best parameter. The signal processing unit 10 may use the heart sound waveform obtained in this way in the calculation of the heartbeat interval, which is the processing to be performed next. In the next frame loop, the signal processing unit 10 similarly may use the number Lbest of bases and adjacent numbers of bases $[L_{best}-num_{base}, ..., L_{best}, ..., L_{best}+num_{base}]$ as parameters of the SSNMF-based noise removal processing in step S22. This allows the signal processing unit 10 to stabilize the heart sound waveform extraction results. The signal processing unit 10 may update the number $L_{best}$ of bases on the basis of the degree of abnormality obtained by SST in the processing to be performed next, namely step S23. During this iterative processing, the signal processing unit 10 can optimize the parameters to be used for noise removal on the basis of the indicator referred to as the degree of abnormality.

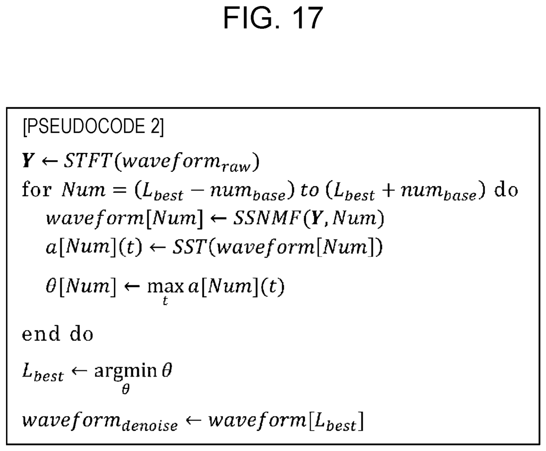

FIG. 17 illustrates an example in which a series of processing steps as described above is summarized as pseudocode. In FIG. 17, $num_{base}$ is a predetermined constant. The signal processing unit 10 repeats the SSNMF processing according to the numerical value of $num_{base}$. The signal processing unit 10 use SST to derive the degree of abnormality a(t) for the waveform obtained by iterative processing, and derives a representative value $\theta$ such as the mean, the minimum, or the median. In this case, the maximum is derived as the representative value $\theta$. After the iterative processing, the signal processing unit 10 updates the number $L_{best}$ of bases to be the number of bases for which the representative value $\theta$ is minimized. The signal processing unit 10 also selects the waveform $waveform_{denoise}$ to be selected for the calculation of the RRI according to the result of the above update. Such processing allows the electronic device 1 to successively optimize the number of bases.

As an example, assume that $num_{base}$ has been set to 5. In this case, the signal processing unit 10 uses five different consecutive integers (for example, 18, 19, 20, 21, 22) as the numbers of bases to be used in SSNMF, and performs SSNMF five times using the five numbers of bases, respectively. The signal processing unit 10 derives the degree of abnormality according to SST five times. At this point, assume that 22 is the number $L_{best}$ of bases for which the lowest degree of abnormality is obtained. In this case, in the next loop of the processing, the signal processing unit 10 repeats the SSNMF and SST processing by using 20, 21, 22, 23, 24 as the numbers of bases. The signal processing unit 10 may use the heart sound waveform extracted using the number $L_{best}$ of bases (equal to 22) to derive the heartbeat interval.

The following describes a usage pattern when operating the electronic device 1 according to an embodiment. The following describes a hardware implementation and a flow of processing when operating the electronic device 1 according to an embodiment.

As illustrated in FIG. 2, in the electronic device 1 according to an embodiment, an audiovisual device 70 may be connected to the communication interface 50 in a wired or wireless manner. The audiovisual device 70 may be any device that allows for a prescribed notification to be issued in the form of audio and/or video to the driver or other occupant on board a moving body such as an automobile, for example, in which the electronic device 1 is installed. The audiovisual device 70 is not necessarily limited to a device that issues a prescribed notification in the form of audio and/or video, and may be any device that allows for a prescribed notification to be issued, such as through tactile feedback, for example. In FIG. 2, the audiovisual device 70 is connected to the communication interface 50, but may also be connected in a wired and/or wireless manner to the signal processing unit 10 and/or the external device 60, for example.

The calculation unit 14 of the signal processing unit 10 illustrated in FIG. 2 may further calculate a coefficient of variation of R-R interval (CVRR) in addition to the processing already described in FIG. 2. The CVRR may mean a coefficient of variation with respect to variation in the R-R interval. The CVRR is an indicator of the degree of variation in the RRI over a fixed time, expressed as a percentage. The CVRR has been reported to correlate with the level of activity in the parasympathetic nervous system activity of a test subject, and is expected to be used to quantify fatigue in the test subject.

The electronic device 1 transmits biological information obtained by calculating the CVRR in the calculation unit 14 to the audiovisual device 70 via the communication interface 50. This enables the test subject to grasp the result of the processing by the electronic device 1. If, for example, the test subject is driving a moving body such as an automobile in which the electronic device 1 is installed and there is a strong possibility of danger due to drowsiness or the like, the electronic device 1 can output visual information and/or auditory information to the test subject from the audiovisual device 70. Visual information outputted by the audiovisual device 70 may be, for example, intense flashing of an indicator light or the like. Auditory information outputted by the audiovisual device 70 may be, for example, a warning sound such as a buzzer sound.

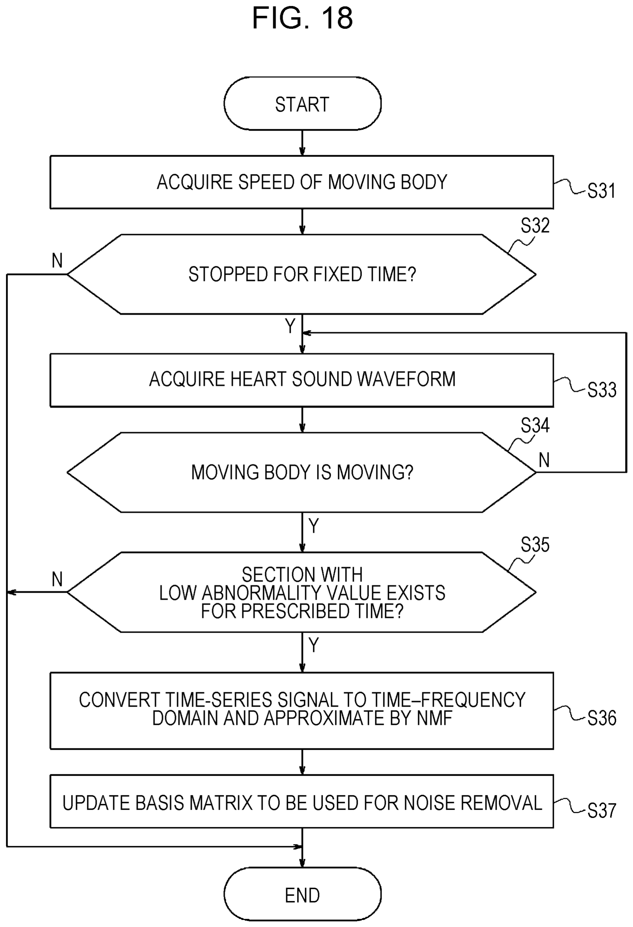

FIG. 18 is a flowchart for describing an example of operations as a driver monitoring system (DMS) by the electronic device 1 according to an embodiment. FIG. 18 may illustrate processing steps for updating the frequency basis matrix to be used in SSNMF by the electronic device 1 according to an embodiment.

In the operations illustrated in FIG. 18, the electronic device 1 according to an embodiment may need a heart sound waveform that is free of noise. For this reason, in the operations illustrated in FIG. 18, the electronic device 1 may need to acquire heart sound at times when vibrations are not being generated in the moving body such as an automobile, for example, in which the electronic device 1 is installed.

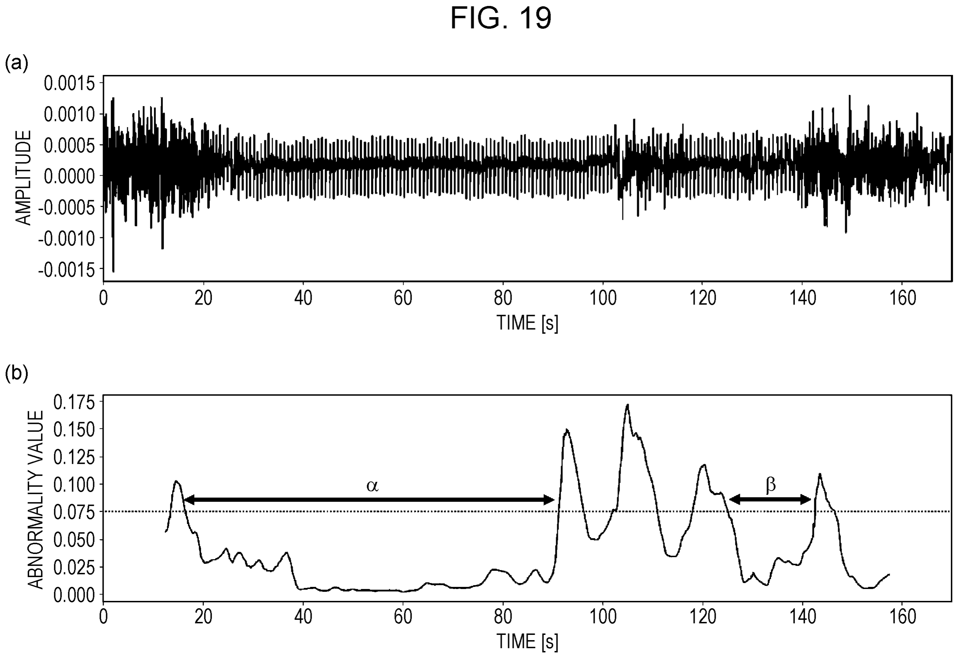

Accordingly, when the operations illustrated in FIG. 19 start, the signal processing unit 10 acquires the speed of travel of the moving body in which the electronic device 1 is installed (step S31). In step S31, the signal processing unit 10 may acquire the vehicle speed of the moving body on the basis of a detection result from an inertial measurement unit (IMU), a detection result regarding tire rotation speed, and/or detection results from any of various devices such as an onboard camera.

In step S32, the signal processing unit 10 determines whether the moving body in which the electronic device 1 is installed has stopped for a fixed time.

As indicated in step S33, the signal processing unit 10 may acquire a heart sound waveform over a fixed time if, during the fixed time, no motion is observed that would result in noise being superimposed due to the movement of the moving body.

As indicated in step S34, the signal processing unit 10 determines whether the moving body in which the electronic device 1 is installed is moving. Upon determining that the moving body is moving, as indicated in step S35, the signal processing unit 10 determines whether a section with a low abnormality value according to SST exists for a prescribed time. The signal processing unit 10 may perform SST-based change detection on a heart sound waveform obtained in the time between when the moving body stops and when the moving body resumes travel. If a section in which the degree of abnormality according to SST falls below a fixed threshold exists for a predetermined time or longer (step S35, Yes), the signal processing unit 10 uses the heart sound waveform of the section in basis matrix creation.

As indicated in step S36, the signal processing unit 10 converts the time-series signal to the time-frequency domain and approximates time-frequency analysis information using non-negative matrix factorization (NMF).

As indicated in step S37, the signal processing unit 10 updates the basis matrix to be used for the removal of noise, and then may end the operations illustrated in FIG. 18.

FIG. 19 illustrates an example of performing abnormality detection on a heart sound waveform acquired by the electronic device 1. FIG. 19 illustrates an example in which the electronic device 1 installed inside a moving body such as an automobile, for example, detects a heart sound waveform of an occupant of the moving body. FIG. 19(a) illustrated in the upper row of FIG. 19 indicates an input waveform. FIG. 19(b) illustrated in the lower row of FIG. 19 indicates the degree of abnormality of the input waveform.

As illustrated in FIG. 19, assume that the moving body in which the electronic device 1 stops at a time around 20 seconds, then resumes travel at a time around 140 seconds. In the example illustrated in FIG. 19, assuming that the degree of abnormality illustrated in FIG. 19(b) has a threshold of 0.075 and the length of time needed for the basis matrix is 40 seconds, the section $\alpha$ illustrated in FIG. 19(b) falls below the threshold of the degree of abnormality for a length of time not less than 40 seconds. In this case, the signal processing unit 10 updates the basis matrix to be used for noise removal. On the other hand, the section $\beta$ illustrated in FIG. 19(b) falls below the threshold of the degree of abnormality for a period of time that is less than 40 seconds. In this case, the signal processing unit 10 does not update the basis matrix to be used for noise removal.

On the other hand, if the section $\beta$ illustrated in FIG. 19(b) had satisfied the conditions regarding the degree of abnormality and the length of time, the section $\beta$ would be the most recent data. Accordingly, the signal processing unit 10 may update the basis matrix to be used for noise removal. The signal processing unit 10 uses the history matrix $H_1$ and the check matrix $H_2$ to obtain the degree of abnormality at the start and end points of the section adopted in the updating of the basis matrix to be used for noise removal. From among any sample points included in the two time series used for formation in the generation of the history matrix $H_1$ and the check matrix $H_2$, the signal processing unit 10 may select one point each from each time-series signal such that the length of the section of the degree of abnormality and a waveform over an equal length of time are obtained.

The signal processing unit 10 converts the heart sound waveform into time-frequency analysis information, such as a spectrogram, using the two selected sample points as the start and end points, and updates the basis matrix F by NMF, as expressed in the following expression (28).

[Math. 28]

$$Y_{target} \simeq FQ \qquad (28)$$

In expression (28) above, $Y_{target}$ indicates the spectrogram represented as in the following expression (29).

[Math. 29]

$$Y_{target}\left(\in \mathbb{R}_{\geq 0}^{\Omega \times T_s}\right) \qquad (29)$$

In expression (28) above, F indicates the basis matrix represented as in the following expression (30).

[Math. 30]

$$F\left(\in \mathbb{R}_{\geq 0}^{\Omega \times K}\right) \qquad (30)$$

In expression (28) above, Q indicates the activation matrix represented as in the following expression (33).

[Math. 31]

$$Q\left(\in \mathbb{R}_{\geq 0}^{K \times T_s}\right) \qquad (31)$$

The signal processing unit 10 uses the update formula expressed in expression (28) above to approximate the matrices expressed in expressions (29) to (31) above. The following describes two basic update formulas.

An update formula corresponding to the cost function based on Euclidean distance can be expressed as in the following expressions (32) and (33).

[Math. 32]

$$f_{\omega,k} = \frac{\sum_{t_s} y_{\omega,t_s} q_{k,t_s}}{\sum_{t_s} q_{k,t_s} \sum_k f_{\omega,k} q_{k,t_s}} f_{\omega,k} \qquad (32)$$

[Math. 33]

$$q_{k,t_s} = \frac{\sum_\omega y_{\omega,t_s} f_{\omega,k}}{\sum_\omega f_{\omega,k} \sum_k f_{\omega,k} q_{k,t_s}} q_{k,t_s} \qquad (3\,3)$$

An update formula corresponding to the cost function based on I-divergence can be expressed as in the following expressions (34) and (35).
[Math. 34]

$$f_{\omega,k} = \frac{\sum_{t_s} \frac{y_{\omega,t_s} q_{k,t_s}}{\sum_k f_{\omega,k} q_{k,t_s}}}{\sum_{t_s} q_{k,t_s}} f_{\omega,k} \qquad (3\,4)$$

[Math. 35]

$$q_{k,t_s} = \frac{\sum_\omega \frac{y_{\omega,t_s} f_{\omega,k}}{\sum_k f_{\omega,k} q_{k,t_s}}}{\sum_\omega f_{\omega,k}} q_{k,t_s} \qquad (3\,5)$$

The signal processing unit 10 selects one variety of the update formulas described above and repeatedly updates a fixed number of iterations, or approximates until a fixed distance is reached.

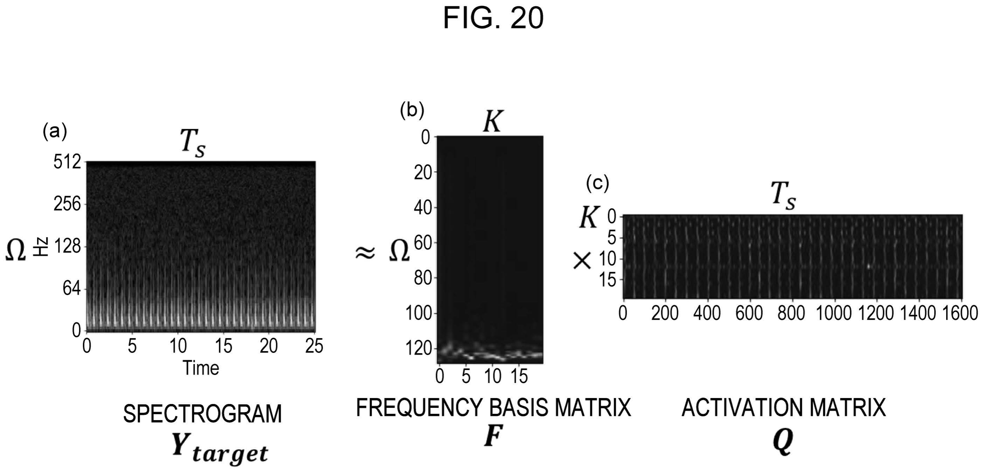

FIG. 20 illustrates an example of the result of NMF-based matrix approximation when a spectrogram of a heart sound waveform is inputted into the signal processing unit 10, analogous to expression (28) above. In FIG. 20, K represents the number of bases.

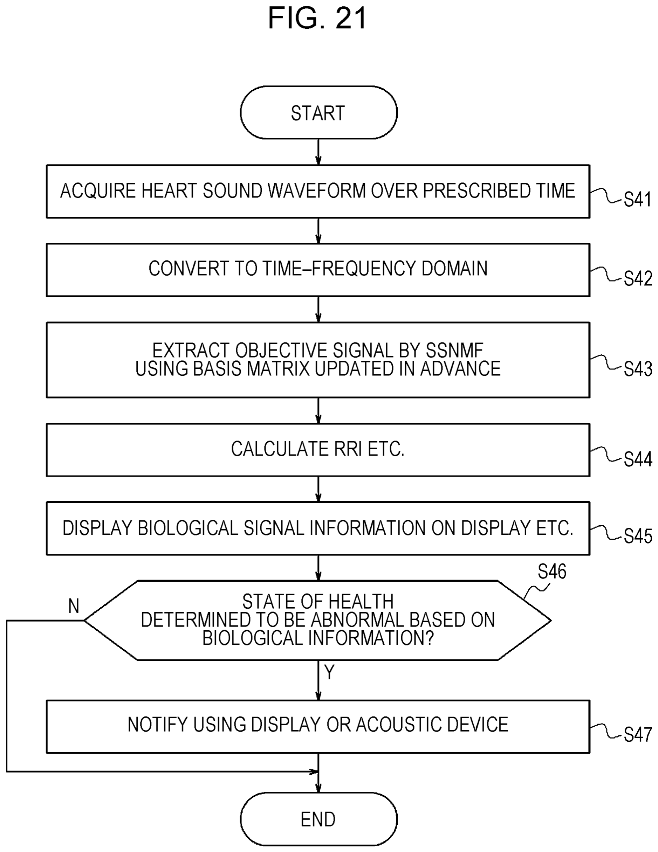

FIG. 21 is a flowchart for describing an example of operations as a driver monitoring system (DMS) by the electronic device 1 according to an embodiment. FIG. 21 is a diagram for describing operations whereby, in an embodiment, the electronic device 1 monitors biological information about a driver and issues a warning in a prescribed case. In FIG. 21, the processing from step S41 to step S44 may correspond to the processing from step S11 to step S16 illustrated in FIG. 12. In step S44, the signal processing unit 10 may calculate the heartbeat interval (RRI) and at least one of the beats per minute (BPM), the HRV, the CVRR, or the like.

In step 45, the signal processing unit 10 presents (displays) information based on a detected biological signal on the audiovisual device 70, which may be a display, for example. As described above, the signal processing unit 10 may also present information based on the detected biological signal as auditory information and/or tactile information from the audiovisual device 70.

In step S46, the signal processing unit 10 may determine whether the test subject is in an abnormal state of health on the basis of the detected biological signal. Upon estimating that there is a strong possibility of a dangerous situation given the state of health of the test subject on the basis of the detected biological signal, the signal processing unit 10 may output a buzzer sound, intense flashing of an indicator light, and/or the like from the audiovisual device 70 to leave the subject in potential danger (step S47).

In this way, in an embodiment, the electronic device 1 is provided with a signal processing unit 10 that detects a subject of monitoring on the basis of a transmission signal transmitted as a transmission wave and a received signal received as a reflected wave resulting from the transmission wave being reflected by the subject of monitoring. The signal processing unit 10 generates first time-frequency analysis information Y1 on the basis of the transmission wave and the reflected wave. The signal processing unit 10 performs SSNMF to calculate the first time-frequency analysis information Y1 as Y1 = FG + HU using the frequency basis matrices F and H and the activation matrices G and U, calculate the frequency basis matrix F as a frequency basis matrix obtained from a reference heart sound waveform obtained in advance, and calculate the activation matrix G using a prescribed cost function. The signal processing unit 10 generates a heart sound waveform of the subject of monitoring on the basis of the product FG of the frequency basis matrix F and the activation matrix G.

In the electronic device 1 according to an embodiment, the signal processing unit 10 may execute processing like the following, taking L to be the number of basis vectors of the frequency basis matrices F and H. That is, for a heart sound waveform generated on the basis of the product FG of the frequency basis matrix F and the activation matrix G, the signal processing unit 10 may also generate the heart sound waveform again using the number L of basis vectors for which the degree of abnormality $\alpha$ is minimized according to singular spectrum transformation. In this case, the degree of abnormality $\alpha(t)$ may be defined as in expression (27) above.

In the electronic device 1 according to an embodiment, the signal processing unit 10 may also generate second time-frequency analysis information Y2 with respect to the heart sound waveform of an occupant when the motion of a moving body carrying the occupant is at or below a prescribed level. In this case, the signal processing unit 10 may also update the basis matrix F by NMF using a cost function by representing the second time-frequency analysis information Y2 as Y2 = FQ, that is, as the product of the basis matrix F and an activation matrix Q.

In the electronic device 1 according to an embodiment, the motion of the moving body carrying the occupant may be considered at or below the prescribed level if the speed of the moving body carrying the occupant is at or a below a prescribed level. The occupant may also be the same person as the subject of monitoring. The electronic device 1 may also be installed inside the moving body.

As described above, according to an embodiment, the electronic device 1 can measure vibration on the body surface of a test subject by applying signal processing to a reflected wave (received wave) obtained from a radar. As a result, according to an embodiment, the electronic device 1 can well acquire a heart sound waveform of a human being or an animal. According to an embodiment, the electronic device 1 can use multivariate analysis to detect the presence or absence of noise, including road noise, superimposed on the acquired heart sound waveform. According to an embodiment, if noise is included in the acquired heart sound waveform, the electronic device 1 can separate the heart sound and the noise.

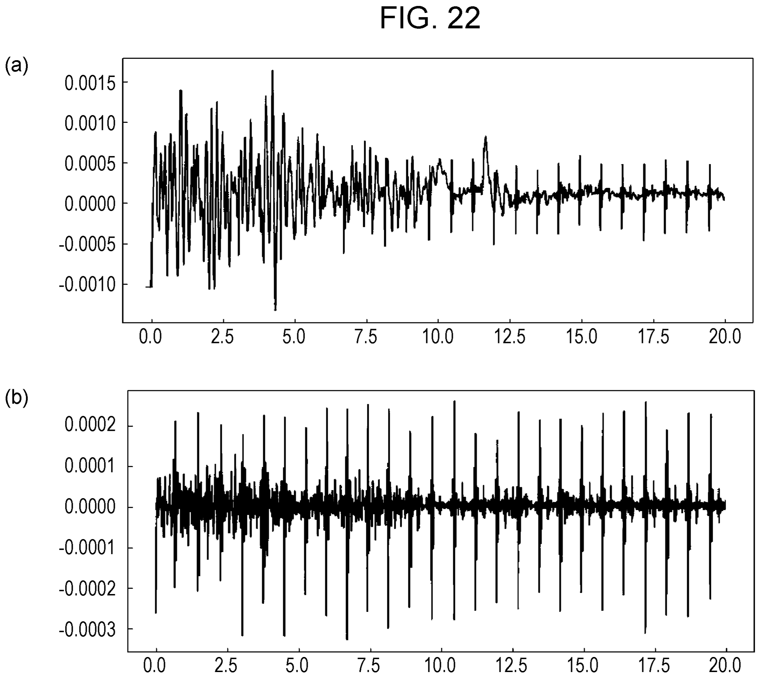

FIG. 22 illustrates an example of the removal of noise from a heart sound waveform by the electronic device 1 according to an embodiment. FIG. 22(a) illustrated in the upper row of FIG. 22 illustrates an example of a heart sound waveform (20 seconds long) before processing to remove noise is performed by the electronic device 1 according to an embodiment. FIG. 22(b) illustrated in the lower row of FIG. 22 illustrates an example of a heart sound waveform (20 seconds long) after processing to remove noise is performed by the electronic device 1 according to an embodiment.

As illustrated in FIG. 22(a), peaks in the heart sound are obscured by road noise in approximately the left half (first 10 seconds) of the heart sound waveform before the noise removal processing is performed. According to an embodiment, the electronic device 1 can achieve noise removal by temporarily converting the heart sound waveform to frequency information and then extracting only the component of the objective signal, as illustrated in approximately the left half (first 10 seconds) of FIG. 22(b).

According to an embodiment, the electronic device 1 does not need an enormous amount of data to remove road noise, as would be the case with deep learning. According to an embodiment, the electronic device 1 can remove noise such as road noise if, for example, a few dozen seconds of the resting heart sound waveform of an individual can be obtained.

According to an embodiment, as illustrated in FIG. 19, for example, the electronic device 1 can perform SST such that noise superimposed on the original waveform illustrated in FIG. 19(a) is detected as illustrated in FIG. 19(b). For example, in an embodiment, if the degree of abnormality is high according to SST, the electronic device 1 can determine that there is a strong possibility that noise is included. According to an embodiment, the electronic device 1 can pick a heart sound waveform to serve as supervisory data for use in road noise removal, re-tune optimal hyperparameters, and/or the like on the basis of the magnitude of the degree of abnormality obtained by performing SST.

(Other embodiments)

The following describes other embodiments.

In the embodiment described above, the electronic device 1 is assumed to remove noise from a heart sound waveform acquired by a millimeter-wave radar, for example. However, in an embodiment, the electronic device 1 ultimately achieves estimation of the heartbeat interval by capturing the vibration of pulsations in human beings and animals transmitted to the body surface. Consequently, in another embodiment, the electronic device 1 may also remove noise from a waveform obtained from a device, such as an acceleration sensor, for example, that can measure vibration at the installation position thereof.

In another embodiment, the electronic device 1 may not only remove noise from a waveform of the pulsations of a human or animal heartbeat, but also similarly remove noise from a human or animal respiratory waveform. Respiration tends to have greater displacement variation affecting the body surface than heartbeat, and the displacement variation tends to be more conspicuous. Accordingly, in another embodiment, the electronic device 1 can acquire a human or animal respiratory waveform more easily than a waveform of heartbeat. In an embodiment of the present disclosure, the electronic device 1 may remove noise due to the body motion involving motion of the head, eyelid, eyeball, pupil, foot, hand, and/or mouth of a human being or an animal.

As described above, in an embodiment, the electronic device 1 may be installed in all types of moving bodies that move on public roads, for example. However, in another embodiment, the electronic device 1 not only may be applied to moving bodies that move on public roads, such as automobiles, but may also be applied similarly in environments where noise is thought to be generated, such as aircraft, ships, or trains, for example. In another embodiment, the electronic device 1 may also be applied similarly to noise generated by physical activity by a test subject in an indoor environment. In an embodiment, the electronic device 1 may be applied to heartbeat measurement and/or noise removal for a test subject riding a motorcycle, a bus, a bicycle, a truck, a construction vehicle, or a tractor, for example.

In another embodiment, the method for evaluating noise removal performance according to degree of abnormality performed in step S23 of FIG. 13 may also be applied to pick a low-noise heart sound waveform in the acquisition of a heart sound waveform in step S33 of FIG. 18. This processing may be important in the acquisition of an appropriate frequency basis matrix in SSNMF used for noise removal.

In another embodiment, the method for evaluating noise removal performance according to degree of abnormality performed in step S23 of FIG. 13 is also applicable in a case where the SSNMF processing to be performed in step S43 of FIG. 21 is omitted when the degree of noise is relatively minor. Omitting the SSNMF processing in this way may reduce the computational processing load. If the noise is relatively slight, the processing to be performed in step S43 of FIG. 21 may also remove noise by naive NMF rather than SSNMF.

In another embodiment, the update formula to be used in the SSNMF processing to be performed in step S22 of FIG. 13 is not limited to the formulas described above, and another valid update formula may also be used. For example, in another embodiment, the electronic device 1 may use an update formula with the added constraint that the two frequency basis matrices are mutually orthogonal, as expressed in the following expression (36).

[Math. 36]

$$\min_{H}\left\|\boldsymbol{F}^{T}\boldsymbol{H}\right\|_{2}^{2} \quad (36)$$

As another example, in another embodiment, the electronic device 1 may use an update formula with the added constraint that the Kullback-Leibler (KL) distance between the two frequency basis matrices is maximized, as expressed in the following expression (37).

[Math. 37]

$$\max_{h_{\omega,l}} \sum_{\omega,k,l}\left\{ f_{\omega,k}\log\frac{f_{\omega,k}}{h_{\omega,l}} - \left(f_{\omega,k} - h_{\omega,l}\right)\right\} \quad (37)$$

In another embodiment, the electronic device 1 may execute more precise signal separation by using an update formula that accounts for constraints like those of expressions (36) and (37) above.

In the embodiment described above, the observation matrix to be applied to SSNMF and NMF is assumed to be a spectrogram as an example. However, in another embodiment, time-frequency analysis information obtained by a wavelet transform or a derivative thereof may also be used instead of a spectrogram.

In another embodiment, if the computational load of detecting abnormalities in time-series signals by SST makes real-time processing difficult, processing speed may be improved by downsampling to a degree that does not cause the heart sound waveform to collapse.

In the electronic device 1 illustrated in FIG. 2, the signal processing unit 10 is described as being provided with functional units such as the heartbeat extraction unit 13 and the calculation unit 14. However, in an embodiment, the processing performed by the heartbeat extraction unit 13 and/or the calculation unit 14 may also be performed by an external computer, processor, or the like.

As described above, in an embodiment, the electronic device 1 uses, for example, a millimeter-wave sensor provided with a plurality of transmitting antennas and a plurality of receiving antennas to detect weak vibrations such as a heartbeat. In an embodiment, when the subject is not detected, the electronic device 1 detects the body motion of the subject while varying the transmission phase of the antennas to create a beamforming pattern of the transmitting antennas. On the other hand, in an embodiment, once the body motion of the subject is detected, the electronic device 1 carries out beamforming in the direction of the body motion to detect the heartbeat. In this way, according to an embodiment, the electronic device 1 can improve the signal quality by automatically detecting the direction of a human body. Consequently, according to an embodiment, the electronic device 1 can improve the heartbeat detection precision and/or detection range. Thus, according to an embodiment, the electronic device 1 can detect the heartbeat of a human being with high precision.

The present disclosure has been described on the basis of the drawings and examples, but note that a person skilled in the art could easily make various variations or revisions on the basis of the present disclosure. Consequently, it should be understood that these variations or revisions are included in the scope of the present disclosure. For example, the functions and the like included in each functional unit may be rearranged in logically non-contradictory ways. Multiple functional units or the like may be combined into one, or a functional unit may be divided. Each embodiment according to the present disclosure described above is not limited to being carried out exactly according to each embodiment as described, and may be carried out by combining features or omitting some features, as appropriate. In other words, the content of the present disclosure enables a person skilled in the art to make various variations and revisions on the basis of the present disclosure. Therefore, these variations and revisions are included in the scope of the present disclosure. For example, in each embodiment, each functional unit, means, step, and the like can be added to another embodiment or replaced by each functional unit, means, step, and the like of another embodiment in logically non-contradictory ways. In each embodiment, multiple functional units, means, steps, and the like can be combined into one, or each functional unit, means, step, and the like can be divided. Each embodiment according to the present disclosure described above is not limited to being carried out exactly according to each embodiment as described, and can be carried out by combining features or omitting some features, as appropriate.

The embodiments described above are not limited solely to embodiments of the electronic device 1. For example, the embodiments described above may also be carried out as a method of controlling a device like the electronic device 1. Furthermore, the embodiments described above may also be carried out as a program to be executed by a device like the electronic device 1, for example, or as a storage medium or recording medium in which the program is recorded.

In the embodiments described above, the electronic device 1 is described as including components such as the transmitting antenna 24 and the receiving antenna 31 that form what is called a radar sensor. However, in an embodiment, the electronic device may be carried out as a configuration like the signal processing unit 10, for example. In this case, the signal processing unit 10 may be carried out as a unit having functions for processing signals handled by the transmitting antenna 24, the receiving antenna 31, and the like.

REFERENCE SIGNS

    1    electronic device
    10    signal processing unit
    11    signal generation processing unit
    12    received signal processing unit
    13    heartbeat extraction unit
    14    calculation unit
    21    transmission DAC
    22    transmission circuit
    23    millimeter-wave transmission circuit
    24    transmitting antenna
    31    receiving antenna
    32    mixer
    33    reception circuit
    34    reception ADC
    50    communication interface
    60    external device
    70    audiovisual device

**Claims**

1. An electronic device comprising a signal processing unit configured to detect a subject of monitoring on the basis of a transmission signal transmitted as a transmission wave and a received signal received as a reflected wave resulting from the transmission wave being reflected by the subject of monitoring,
wherein the signal processing unit is configured to
generate a heart sound waveform of the subject of monitoring by using basis information pertaining to a heart sound waveform obtained in advance to perform non-negative matrix factorization of time-frequency analysis information calculated on the basis of the transmission wave and the reflected wave.

2. An electronic device comprising a signal processing unit configured to detect a subject of monitoring on the basis of a transmission signal transmitted as a transmission wave and a received signal received as a reflected wave resulting from the transmission wave being reflected by the subject of monitoring,
wherein the signal processing unit is configured to

    generate first time-frequency analysis information Y1 on the basis of the transmission wave and the reflected wave,

perform semi-supervised non-negative matrix factorization to calculate the first time-frequency analysis information Y1 as Y1 = FG + HU using frequency basis matrices F and H and activation matrices G and U, calculate the frequency basis matrix F as a frequency basis matrix obtained from the reference heart sound waveform obtained in advance, and calculate the activation matrix G using a prescribed cost function; and
generate the heart sound waveform of the subject of monitoring on the basis of the product FG of the frequency basis matrix F and the activation matrix G.

3. The electronic device according to claim 2, wherein

provided that L is the number of basis vectors of the frequency basis matrices F and H,
the signal processing unit is configured to
define a degree of abnormality $\alpha(t)$ according to singular spectrum transformation as

$$ a(t) = 1 - \left| U_1^{(r)^{\mathsf{T}}} U_2^{(r)} \right|_2 $$

for the heart sound waveform generated on the basis of the product FG of the frequency basis matrix F and the activation matrix G, and
generate the heart sound waveform again using the number L of basis vectors for which the degree of abnormality $\alpha$ is minimized.

4. The electronic device according to claim 2, wherein
the signal processing unit is configured to

generate second time-frequency analysis information Y2 with respect to a heart sound waveform of an occupant when the motion of a moving body carrying the occupant is at or below a prescribed level, and
update the basis matrix F by non-negative matrix factorization using a cost function by representing the second time-frequency analysis information Y2 as Y2 = FQ, that is, as the product of the basis matrix F and an activation matrix Q.

5. The electronic device according to claim 4, wherein
the motion of the moving body carrying the occupant is considered at or below the prescribed level if the speed of the moving body is at or a below a prescribed level.

6. The electronic device according to claim 4, wherein
the occupant is the same person as the subject of monitoring.

7. The electronic device according to claim 4, wherein
the electronic device is installed inside the moving body.

8. A method of controlling an electronic device, the method comprising:

detecting a subject of monitoring on the basis of a transmission signal transmitted as a transmission wave and a received signal received as a reflected wave resulting from the transmission wave being reflected by the subject of monitoring;
generating first time-frequency analysis information Y1 on the basis of the transmission wave and the reflected wave;
performing semi-supervised non-negative matrix factorization to calculate the first time-frequency analysis information Y1 as Y1 = FG + HU using frequency basis matrices F and H and activation matrices G and U, calculate the frequency basis matrix F as a frequency basis matrix obtained from the reference heart sound waveform obtained in advance, and calculate the activation matrix G using a prescribed cost function; and
generating the heart sound waveform of the subject of monitoring on the basis of the product FG of the frequency basis matrix F and the activation matrix G.

9. A program causing an electronic device to execute a process comprising:

detecting a subject of monitoring on the basis of a transmission signal transmitted as a transmission wave and a received signal received as a reflected wave resulting from the transmission wave being reflected by the subject of monitoring;

generating first time-frequency analysis information Y1 on the basis of the transmission wave and the reflected wave;

performing semi-supervised non-negative matrix factorization to calculate the first time-frequency analysis information Y1 as Y1 = FG + HU using frequency basis matrices F and H and activation matrices G and U, calculate the frequency basis matrix F as a frequency basis matrix obtained from the reference heart sound waveform obtained in advance, and calculate the activation matrix G using a prescribed cost function; and

generating the heart sound waveform of the subject of monitoring on the basis of the product FG of the frequency basis matrix F and the activation matrix G.

# FIG. 1

# FIG. 2

# FIG. 3

EP 4 703 766 A1

# FIG. 4

SUBFRAME 1

SUBFRAME N

# FIG. 5

# FIG. 6

FIG. 7

# FIG. 8

FIG. 9

# FIG. 10

EP 4 703 766 A1

# FIG. 11

START

↓

PROCESS RECEIVED SIGNAL — S110

↓

EXTRACT SIGNAL SOURCE — S120

↓

CONVERT TO VIBRATION WAVEFORM — S130

↓

EXTRACT VIBRATION DATA — S140

↓

CALCULATE RRI — S150

↓

CALCULATE HRV — S160

↓

END

# FIG. 12

```
        ┌─────────────┐
        │    START    │
        └──────┬──────┘
               │
               ▼
   ┌──────────────────────────┐
   │  PROCESS RECEIVED SIGNAL │ ～ S11
   └──────────┬───────────────┘
               │
               ▼
   ┌──────────────────────────┐
   │   EXTRACT SIGNAL SOURCE  │ ～ S12
   └──────────┬───────────────┘
               │
               ▼
   ┌──────────────────────────┐
   │ CONVERT TO VIBRATION WAVEFORM │ ～ S13
   └──────────┬───────────────┘
               │
               ▼
   ┌──────────────────────────┐
   │   EXTRACT VIBRATION DATA │ ～ S14
   └──────────┬───────────────┘
               │
               ▼
   ┌──────────────────────────┐
   │       REMOVE NOISE       │ ～ S15
   └──────────┬───────────────┘
               │
               ▼
   ┌──────────────────────────┐
   │  CALCULATE RRI, HRV, ETC.│ ～ S16
   └──────────┬───────────────┘
               │
               ▼
        ┌─────────────┐
        │     END     │
        └─────────────┘
```

# FIG. 13

```
        ┌─────────────┐
        │    START    │
        └─────────────┘
               │
               ▼
┌──────────────────────────────┐
│      ANALYZE FREQUENCY       │─── S21
└──────────────────────────────┘
               │
               ▼
┌──────────────────────────────┐
│   PERFORM SEMI-SUPERVISED    │─── S22
│ NON-NEGATIVE MATRIX FACTORIZATION │
└──────────────────────────────┘
               │
               ▼
┌──────────────────────────────┐
│     SELECT BEST WAVEFORM     │─── S23
│  BY SINGULAR SPECTRUM TRANSFORM │
└──────────────────────────────┘
               │
               ▼
        ┌─────────────┐
        │     END     │
        └─────────────┘
```

# FIG. 14

[PSEUDOCODE 1]

$Y \leftarrow STFT(waveform_{raw})$

$G_{[0]} \leftarrow random(K, T)$

$H_{[0]} \leftarrow random(\Omega, L)$

$U_{[0]} \leftarrow random(L, T)$

for $iter = 1 \, to \, IterNum$ do

   $G_{[iter]} \leftarrow Func_G(G_{[iter-1]})$

   $H_{[iter]} \leftarrow Func_H(H_{[iter-1]})$

   $U_{[iter]} \leftarrow Func_U(U_{[iter-1]})$

   if $D(Y|FG + HU) < \epsilon$

      break

   end if

end do

$waveform_{denoise} \leftarrow ISTFT(FG)$

# FIG. 15

(a)

(b)

(c)

TIME SIGNAL
(TIME DOMAIN)

STFT

INVERSE STFT

INVERSE STFT

(d)

(e)

(f)

SPECTROGRAM
(TIME–FREQUENCY
DOMAIN)

$$Y \simeq FG + HU$$

EP 4 703 766 A1

FIG. 16

# FIG. 17

[PSEUDOCODE 2]

$Y \leftarrow STFT(waveform_{raw})$

for $Num = (L_{best} - num_{base})\ to\ (L_{best} + num_{base})$ do

$waveform[Num] \leftarrow SSNMF(Y, Num)$

$a[Num](t) \leftarrow SST(waveform[Num])$

$\theta[Num] \leftarrow \max_{t} a[Num](t)$

end do

$L_{best} \leftarrow \underset{\theta}{\operatorname{argmin}}\ \theta$

$waveform_{denoise} \leftarrow waveform[L_{best}]$

# FIG. 18

START

ACQUIRE SPEED OF MOVING BODY — S31

STOPPED FOR FIXED TIME? — S32

N

Y

ACQUIRE HEART SOUND WAVEFORM — S33

MOVING BODY IS MOVING? — S34

N

Y

SECTION WITH
LOW ABNORMALITY VALUE EXISTS
FOR PRESCRIBED TIME? — S35

N

Y

CONVERT TIME-SERIES SIGNAL TO TIME–FREQUENCY
DOMAIN AND APPROXIMATE BY NMF — S36

UPDATE BASIS MATRIX TO BE USED FOR NOISE REMOVAL — S37

END

# FIG. 19

(a)

(b)

EP 4 703 766 A1

FIG. 20

(a)

$T_s$

512
256
$\Omega$ Hz 128
64
0

0   5   10   15   20   25
Time

SPECTROGRAM
$Y_{target}$

(b) $K$

0
20
40
$\approx$ $\Omega$ 60
80
100
120

0  5  10  15

FREQUENCY BASIS MATRIX
$F$

(c) $T_s$

$K$ 0
5
$\times$ 10
15

0   200  400  600  800  1000 1200 1400 1600

ACTIVATION MATRIX
$Q$

EP 4 703 766 A1

# FIG. 21

```
START
```

ACQUIRE HEART SOUND WAVEFORM OVER PRESCRIBED TIME — S41

CONVERT TO TIME–FREQUENCY DOMAIN — S42

EXTRACT OBJECTIVE SIGNAL BY SSNMF
USING BASIS MATRIX UPDATED IN ADVANCE — S43

CALCULATE RRI ETC. — S44

DISPLAY BIOLOGICAL SIGNAL INFORMATION ON DISPLAY ETC. — S45

S46
STATE OF HEALTH
DETERMINED TO BE ABNORMAL BASED ON
BIOLOGICAL INFORMATION?
N

Y

NOTIFY USING DISPLAY OR ACOUSTIC DEVICE — S47

```
END
```

## FIG. 22

(a)

(b)

## INTERNATIONAL SEARCH REPORT

| | International application No. |
|---|---|
| | **PCT/JP2024/014446** |

**A. CLASSIFICATION OF SUBJECT MATTER**

*G01S 13/52*(2006.01)i; *A61B 5/11*(2006.01)i; *G01S 13/34*(2006.01)i
FI:   G01S13/52; A61B5/11 110; G01S13/34

According to International Patent Classification (IPC) or to both national classification and IPC

**B. FIELDS SEARCHED**

Minimum documentation searched (classification system followed by classification symbols)

G01S7/00-G01S7/42; G01S13/00-G01S13/95; A61B5/11

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2023/016948 A1 (IEE INTERNATIONAL ELECTRONICS & ENGINEERING S.A.) 16 February 2023 (2023-02-16) | 1 |
| | paragraphs [0052]-[0085], fig. 1-9 | |
| Y | paragraphs [0052]-[0085], fig. 1-9 | 2, 4-9 |
| Y | JP 2017-151228 A (YAMAHA CORPORATION) 31 August 2017 (2017-08-31) paragraphs [0019]-[0023] | 2, 4-9 |
| A | WO 2016/051565 A1 (HITACHI, LTD.) 07 April 2016 (2016-04-07) entire text, all drawings | 1-9 |
| A | WO 2021/166243 A1 (NEC CORPORATION) 26 August 2021 (2021-08-26) entire text, all drawings | 1-9 |
| A | US 2019/0348059 A1 (ACCUSONUS, INC.) 14 November 2019 (2019-11-14) entire text, all drawings | 1-9 |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **05 June 2024** | **25 June 2024** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

## INTERNATIONAL SEARCH REPORT
### Information on patent family members

| International application No. |
| --- |
| **PCT/JP2024/014446** |

| Patent document cited in search report | | | Publication date (day/month/year) | Patent family member(s) | | | Publication date (day/month/year) |
| --- | --- | --- | --- | --- | --- | --- | --- |
| WO | 2023/016948 | A1 | 16 February 2023 | LU | 500923 | B1 | |
| JP | 2017-151228 | A | 31 August 2017 | (Family: none) | | | |
| WO | 2016/051565 | A1 | 07 April 2016 | (Family: none) | | | |
| WO | 2021/166243 | A1 | 26 August 2021 | (Family: none) | | | |
| US | 2019/0348059 | A1 | 14 November 2019 | (Family: none) | | | |

Form PCT/ISA/210 (patent family annex) (July 2022)

**EP 4 703 766 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2023072698 A
- JP 2015145754 A
- JP 2017151872 A
- JP 2018136368 A